# EUROPEAN PATENT APPLICATION

(11) **EP 4 289 818 A1**
(43) Date of publication of application: **13.12.2023**
(21) Application number: 21924424.1
(22) Date of filing: 17.12.2021
(51) Int. Cl.: C07C 403/14, C07C 403/12, C07C 403/08

(54) **TOTAL SYNTHESIS METHOD FOR VITAMIN A, DERIVATIVE AND DEUTERATED COMPOUND THEREOF**

(30) Priority: 08.02.2021 CN 202110170708
(71) Applicant: Fudan University, Shanghai 200433 (CN)
(72) Inventor: MA, Shengming, Shanghai 200433 (CN); WANG, Weiyi, Shanghai 200433 (CN); YU, Yibo, Shanghai 200433 (CN); QIAN, Hui, Shanghai 200433 (CN)
(74) Representative: Barker Brettell LLP
(86) International application number: PCT/CN2021/139157
(87) International publication number: WO 2022/166431

(57) **Abstract**

Disclosed in the present invention is a total synthesis method for vitamin A and derivative thereof and deuterated compound thereof. β-cyclocitral is used as a starting material to produce alkenyl boronate, then the alkenyl boronate (or hydrolyzed alkenyl boric acid) is subjected to a series of reactions to produce retinal or retinal with substituent, and the retinal or retinal with substituent is further subjected to a reduction reaction to obtain vitamin A or vitamin A with substituent; the vitamin A or vitamin A with substituent is subjected to an esterification reaction to obtain vitamin A ester or vitamin A ester with substituent; or said retinal or retinal with substituent is subjected to an oxidation reaction to obtain vitamin A acid or vitamin A acid with substituent. When deuterated allenol is used, deuterated vitamin A and derivative thereof are obtained. The present invention has the advantages of short synthetic route, simple operation, readily available raw materials and reagents, and modularization and divergence, and can synthesize the deuterated vitamin A and derivative thereof which are difficult to synthesize in the prior art.

## Description

### TECHNICAL FIELD

The invention belongs to the technical field of chemical synthesis, and relates to a total synthesis method of vitamin A and derivative thereof and deuterated compound thereof.

### BACKGROUND OF THE INVENTION

Vitamin A (retinol) is an important nutrient and a fat-soluble vitamin necessary for the human body to maintain normal metabolism and function. It has various physiological functions such as promoting growth, reproduction, maintaining vision and epithelial tissues. Important derivatives of vitamin A include retinal and retinoic acid, both of which have similar physiological functions and pharmacological activities. Although vitamin A can be extracted from animal tissues, the steps are complicated and the cost is high, so almost all commercial vitamin A is produced by chemical synthesis. Due to the instability of vitamin A, the main vitamin A products at present are vitamin A acetate, vitamin A palmitate, etc. (Ref: (a) Parker, G. L.; Smith, L. K.; Baxendale, I. R. Tetrahedron 2016, 72, 1645-1652. (b) Eggersdorfer, M.; Laudert, D.; Létinois, U.; McClymont, T.; Medlock, J.; Netscher, T.; Bonrath, W. Angew. Chem. Int. Ed. 2012, 51, 12960-12990.)

Vitamin A is a highly capital and technology-intensive product. At present, there are two main routes for the industrial synthesis of vitamin A in the world (as shown in the following formula (B), only the key steps are drawn in the figure): Hoffmann-La Roche route (C14+ C6) and the BASF route (C15+C5).

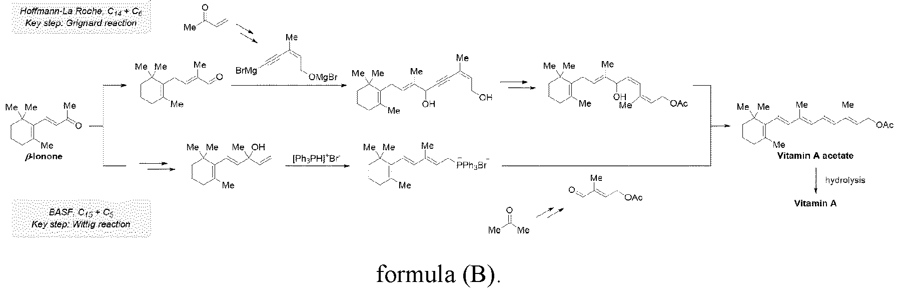

A key step in the Hoffmann-La Roche route is the Grignard reaction. This route is the main synthesis route adopted by vitamin A manufacturers in the world, and the process is relatively mature, but the disadvantage is that it involves many raw materials and auxiliary materials, the route is long, and the equipment is complicated. The key step of the BASF route is the Wittig reaction. This route has fewer reaction steps and a shorter route, but has high requirements for process and equipment, especially the core step Wittig reaction, and the treatment process of by-products is complicated. Although the industrial production of vitamin A has a history of more than 70 years, these industrial synthesis routes also have their own shortcomings, and new routes need to be improved or developed. At present, the technical barriers to vitamin A production are still high, and the research on new synthetic methods is still active.

Deuterated compounds are widely used in drug research (Ref: Pirali, T.; Serafini, M.; Cargnin, S.; Genazzani, A. A. J. Med. Chem. 2019, 62, 5276-5297.), mechanism research (Ref: Gomez-Gallego, M.; Sierra, M. A. Chem. Rev. 2011, 111, 4857-4963.), biochemical research (Ref: Atzrodt, J.; Derdau, V.; Kerr, W. J.; Reid, M. Angew. Chem. Int. Ed. 2018, 57, 1758-1784.). Since the first deuterated drug Austedo (Ref: Schmidt, C. Nat. Biotechnol. 2017, 35, 493-494.) was approved by the U.S. Food and Drug Administration (FDA) in 2017, the research on deuterated drugs has developed rapidly (Ref: (a) Atzrodt, J.; Derdau, V.; Fey, T.; Zimmermann, J. Angew. Chem. Int. Ed. 2007, 46, 7744-7765. (b) Atzrodt, J.; Derdau, V.; Kerr, W. J.; Reid, M. Angew. Chem. Int. Ed. 2018, 57, 3022-3047. (c) Valero, M.; Derdau, V. J. Label. Compd. Radiopharm. 2019, 1-15.). Among them, the deuterated drug ALK-001 (deuterated vitamin A acetate) is used for the treatment of Stargardt disease and is currently in phase II clinical trial. At present, the synthesis of deuterated vitamin A and derivative thereof is very complicated, and only a few structures of deuterated vitamin A and derivative thereof have been reported. For the inert methyl groups on the polyene structure of vitamin A and derivative thereof, there is no synthetic method that can flexibly control the number of deuterium atoms on the methyl group. Moreover, this controllable and divergent deuterium synthesis method is also a great challenge in the field of organic synthesis.

### SUMMARY OF THE INVENTION

In order to solve the deficiencies in the prior art, the purpose of the present invention is to provide a more concise, easier to operate, modular total synthesis method of vitamin A and derivative thereof and deuterated compound thereof. When deuterated raw materials are used, a variety of deuterated vitamin A and derivative thereof which are difficult to be synthesized by the prior art can be synthesized in a modular manner. The method of the present invention has the advantages of short synthesis route, simple operation, readily available raw materials and reagents, modularity and divergence, and can synthesize various deuterated vitamin A and derivative thereof which are difficult to synthesize in the prior art.

The invention provides a total synthesis method of vitamin A and derivative thereof and deuterated compound thereof, said total synthesis method comprises the following steps:
(1) the alkenyl boronate shown in **S2** is produced from β-cyclocitral shown in **S1** and bis[(pinacol)boryl]methane under the action of lithium tetramethylpiperidide in the first organic solvent;
(2) the 2-alkenal shown in **S4** is produced from the alkenyl boronate shown in **S2** and the allenol shown in **5** under the action of rhodium catalyst, copper catalyst, alkali, water, air (or oxygen) in the second organic solvent; or, the alkenyl boronate shown in **S2** is first hydrolyzed into the alkenyl boronic acid shown in S3, and then combined with the allenol shown in **5,** under the action of rhodium catalyst, copper catalyst, alkali, air (or oxygen), in the second organic solvent to produce the 2-alkenal shown in **S4;**
(3) the alkenyl boronate shown in **S5** is produced from the 2-alkenal shown in **S4** and bis[(pinacol)boronyl]methane under the action of lithium tetramethylpiperidide in the first organic solvent;
(4) the retinal or the retinal with substituent shown in **1** is produced from the alkenyl boronate shown in **S5** and the allenol shown in **5,** under the action of rhodium catalyst, copper catalyst, alkali, water, air (or oxygen) in the second organic solvent;
(5) the retinal or the retinal with substituent shown in **1** is subjected to a reduction reaction to produce the vitamin A or the vitamin A with substituents shown in **2;**
(6) the vitamin A or the vitamin A with substituent shown in **2** is subjected to an esterification reaction to produce the vitamin A ester or the vitamin A ester with substituent shown in **3;**
(7) the retinal or the retinal with substituent shown in **1** is subjected to an oxidation reaction to produce the vitamin A acid or the vitamin A acid with substituent shown in **4;**

in the above-mentioned step (2) and/or step (4), when using the allenol shown in **5** labeled with hydrogen isotope, finally obtaining the vitamin A or the vitamin derivative labeled with hydrogen isotope;
the synthetic route of said total synthesis method is shown in the following formula (A): 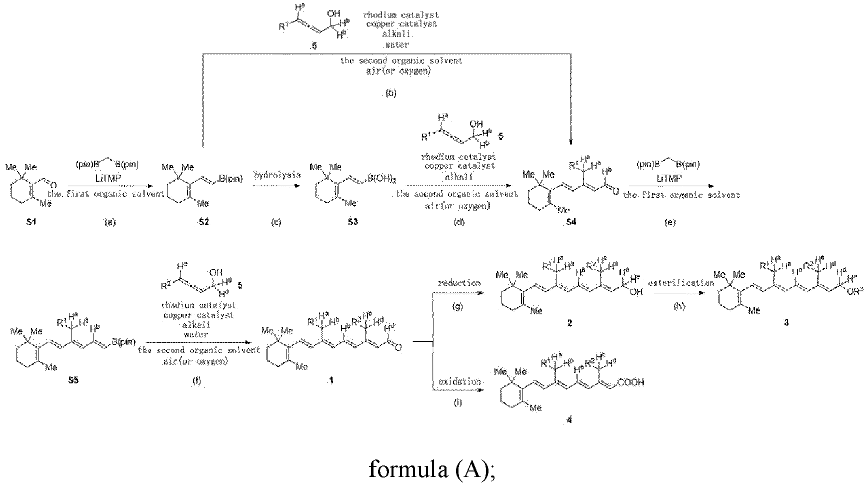
wherein,
   H^{a} is hydrogen, deuterium, tritium;
   R¹ is hydrogen, deuterium, tritium, phenyl, aryl, heterocyclic, hydrocarbyl, hydrocarbyl group with functional group;
   H^{b} is hydrogen, deuterium, tritium;
   H^{c} is hydrogen, deuterium, tritium;
   R² is hydrogen, deuterium, tritium, phenyl, aryl, heterocyclic, hydrocarbyl, hydrocarbyl group with functional group;
   H^{d} is hydrogen, deuterium, tritium;
   H^{e} is hydrogen, deuterium , tritium;
   R³ is an acyl group;
   wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group.

The total synthesis method of the present invention comprises the reaction shown in the reaction formula (a): 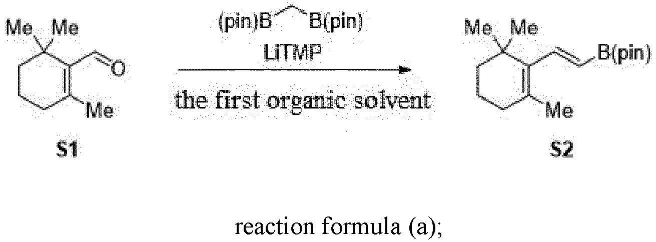
in said reaction formula (a), bis[(pinacol)boronyl]methane reacts with β-cyclocitral (**S1**) under the action of the alkali lithium 2,2,6,6-tetramethylpiperidide (LiTMP) in the first organic solvent to produce compound **S2;** among them, lithium 2,2,6,6-tetramethylpiperidide can be prepared on-site by using 2,2,6,6-tetramethylpiperidine and n-butyllithium;
said reaction shown in formula (a) specifically comprises the following steps:
   (1) protecting the reaction system with inert gas; adding 2,2,6,6-tetramethylpiperidine and the first organic solvent into a dry reaction flask, and adding n-butyllithium reagent at -90~30 °C, then continuing stirring for 15 minutes~3 hours at -90~30 °C, preparing lithium 2,2,6,6-tetramethylpiperidide solution on site; preferably, the reaction temperature is -80~0 °C, and the stirring time is 0.5∼2 hours;
   (2) dissolving bis[(pinacol)boronyl]methane in the first organic solvent, which is added to the lithium 2,2,6,6-tetramethylpiperidide solution at -90~30 °C, and then stirring at -90~30 °C for 1 minute to 2 hours; preferably, adding to the lithium 2,2,6,6-tetramethylpiperidide solution at -20~20 °C, and then continuing stirring for 1~30 minutes at -20~20 °C;
   (3) dissolving β-cyclocitral in the first organic solvent, which is added to the mixture generated in step (2) at -90~30 °C, and then stirring the reaction at -90~30 °C for 0.5∼12 hours; preferably, adding to the mixture generated in step (2) at -80-0 °C, and then stirring the reaction at -80-0 °C for 0.5∼6 hours;
   (4) after the completion of the reaction in step (3), quenching the reaction, and obtaining compound **S2** through extraction, concentration and separation;
said first organic solvent is one or more of tetrahydrofuran, 1,4-dioxane, ether, anisole, methyl tert-butyl ether, ethylene glycol dimethyl ether, ethylene glycol diethyl ether, and toluene; preferably, is tetrahydrofuran.

The molar ratio of said β-cyclocitral (**S1**), lithium 2,2,6,6-tetramethylpiperidide and bis[(pinacol)boryl]methane is 1:(1.0~2.0):(1.0~2.0), preferably is 1:(1.1-1.6):(1.1-1.6); if lithium 2,2,6,6-tetramethylpiperidide solution is prepared on site, the molar ratio of said β-cyclocitral (**S1**), 2,2,6,6-tetramethylpiperidine, n-butyllithium and bis[(pinacol)boryl]methane is 1:(1.0-2.0):(1.0-2.0): (1.0~2.0), preferably, is 1: (1.1-1.6): (1.1-1.6): (1.1-1.6).

The total synthesis method of the present invention comprises the reaction shown in the reaction formula (b): 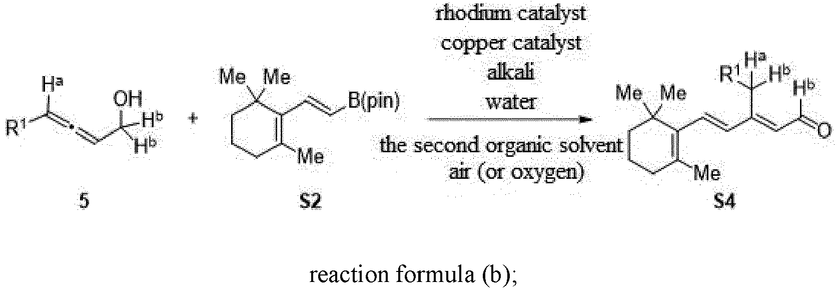 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
preferably, said R¹ is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group.

In said reaction formula (b), allenol **5** and compound **S2** under the action of rhodium catalyst, copper catalyst, alkali, water, air or oxygen in the second organic solvent are reacted to produce compound **S4;**
said reaction shown in the reaction formula (b) specifically comprises the following steps:
   (1) putting rhodium catalyst, alkali, copper catalyst, compound **S2,** allenol **5,** water and the second organic solvent into a dry reaction flask, after plugging the reaction flask with a rubber plug, inserting an air balloon or an oxygen balloon to make the reaction flask in an atmosphere of air or oxygen, and stirring the reaction at -20~60 °C for 4∼96 hours;
   (2) after the completion of the reaction in step (1), filtering the mixture in the reaction flask with a short column of silica gel or diatomaceous earth, washing with the third organic solvent, then concentrating, separating to obtain compound **S4;**
said second organic solvent is one or more of tetrahydrofuran, 1,4-dioxane, toluene, acetonitrile, methanol, ethanol, dichloromethane, ether, anisole, methyl tert-butyl ether, ethylene glycol dimethyl ether, ethylene glycol diethyl ether etc.; preferably, is tetrahydrofuran.

The amount of said second organic solvent refers to the amount of allenol 5 shown in the reaction formula (b) as the basis, the amount of said second organic solvent is 1.0-20.0 mL/mmol; preferably, is 5.0 -10.0 mL/mmol.

Said third organic solvent is one or more of ethyl acetate, ether, methanol, ethanol, dichloromethane, tetrahydrofuran, 1,4-dioxane, acetone, acetonitrile, etc., preferably, is ethyl acetate; the amount of said third organic solvent refers to the amount of allenol **5** shown in the reaction formula (b) as the basis, the amount of said third organic solvent is 1.0~200 mL/mmol, preferably, is 20-100 mL/mmol.

Said rhodium catalyst is any one or more of dichloro(pentamethylcyclopentadienyl)rhodium(III) dimer, tris(acetonitrile)(pentamethylcyclopentadienyl)rhodium(III) bis(hexafluoroantimonate), rhodium(III) acetylacetonate, pentaamminechlororhodium(III) dichloride, trichlorotris(ethylenediamine)rhodium(III), potassium pentachlororhodate(III), sodium hexachlororhodate(III), potassium hexachlororhodate(III), rhodium(III) trichloride, rhodium(III) bromide, rhodium(III) iodide, rhodium(III) sulfate, rhodium(III) nitrate, potassium hexanitrorhodate(III); preferably, is dichloro(pentamethylcyclopentadienyl)rhodium(III) dimer.

Said copper catalyst is any one or more of copper acetate hydrate, copper acetate, copper sulfate hydrate, copper sulfate, copper nitrate hydrate, copper nitrate, copper chloride hydrate, copper chloride, copper bromide, etc.; preferably, is copper acetate monohydrate.

Said alkali is any one or more of sodium acetate, sodium carbonate, sodium bicarbonate, potassium acetate, potassium carbonate, potassium bicarbonate, cesium carbonate, lithium carbonate, magnesium acetate, calcium acetate, etc.; preferably, is sodium acetate.

The molar ratio of said rhodium catalyst, copper catalyst, alkali, compound **S2,** allenol **5,** and water is (0.005∼0.10): (0.005∼1.20): (0∼0.60): (1.0∼3.0): 1.0: (0∼20.0); preferably, is (0.010-0.025):(0.05-0.10):(0.20-0.30):(1.2-2.0):1.0:(2.0-5.0).

The total synthesis method of the present invention comprises the reaction shown in the reaction formula (c): 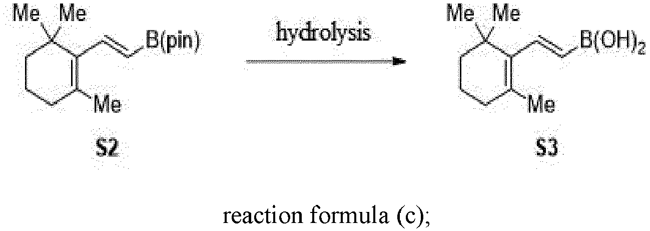 in said reaction formula (c), compound **S2** is hydrolyzed to produce compound **S3.**

The present invention can use any hydrolysis reaction to produce compound **S3;** preferably, compound **S2** is subjected to a hydrolysis reaction under the action of sodium periodate and ammonium acetate in a mixture of acetone and water at 0~40°C to produce compound **S3.**

The total synthesis method of the present invention comprises the reaction shown in the reaction formula (d): 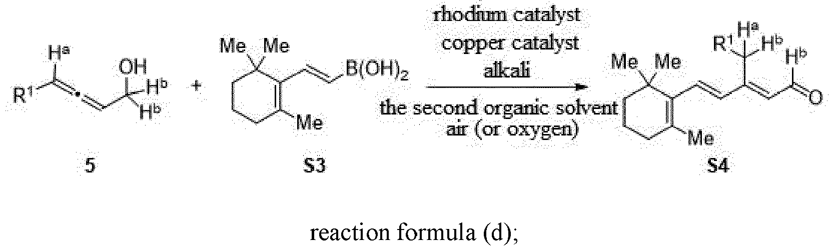 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
preferably, said R¹ is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group.

In said reaction formula (d), allenol **5** and compound **S3** under the action of rhodium catalyst, copper catalyst, alkali, air or oxygen in the second organic solvent are reacted to produce compound **S4;** said compound **S3** can be obtained by the hydrolysis of compound **S2;**
said reaction shown in the reaction formula (d) specifically comprises the following steps:
   (1) putting rhodium catalyst, alkali, copper catalyst, compound **S3,** allenol **5** and the second organic solvent into a dry reaction flask, after plugging the reaction flask with a rubber plug, inserting an air balloon or an oxygen balloon to make the reaction flask in an atmosphere of air or oxygen, and stirring the reaction at -20~60 °C for 4∼96 hours;
   (2) after the completion of the reaction in step (1), filtering the mixture in the reaction flask with a short column of silica gel (or diatomaceous earth), washing with the third organic solvent, then concentrating, separating to obtain compound **S4;**
said second organic solvent is one or more of tetrahydrofuran, 1,4-dioxane, toluene, acetonitrile, methanol, ethanol, dichloromethane, ether, anisole, methyl tert-butyl ether, ethylene glycol dimethyl ether, ethylene glycol diethyl ether etc.; preferably, is tetrahydrofuran.

The amount of said second organic solvent refers to the amount of allenol **5** shown in the reaction formula (d) as the basis, the amount of said second organic solvent is 1.0-20.0 mL/mmol; preferably, is 5.0 -10.0 mL/mmol.

Said third organic solvent is one or more of ethyl acetate, ether, methanol, ethanol, dichloromethane, tetrahydrofuran, 1,4-dioxane, acetone, acetonitrile etc., preferably, is ethyl acetate; the amount of said third organic solvent refers to the amount of allenol **5** shown in the reaction formula (d) as the basis, the amount of said third organic solvent is 1.0~200 mL/mmol, preferably, is 20-100 mL/mmol.

Said rhodium catalyst is any one or more of dichloro(pentamethylcyclopentadienyl)rhodium(III) dimer, tris(acetonitrile)(pentamethylcyclopentadienyl)rhodium(III) bis(hexafluoroantimonate), rhodium(III) acetylacetonate, pentaamminechlororhodium(III) dichloride, trichlorotris(ethylenediamine)rhodium(III), potassium pentachlororhodate(III), sodium hexachlororhodate(III), potassium hexachlororhodate(III), rhodium(III) trichloride, rhodium(III) bromide, rhodium(III) iodide, rhodium(III) sulfate, rhodium(III) nitrate, potassium hexanitrorhodate(III); preferably, is dichloro(pentamethylcyclopentadienyl)rhodium(III) dimer.

Said copper catalyst is any one or more of copper acetate hydrate, copper acetate, copper sulfate hydrate, copper sulfate, copper nitrate hydrate, copper nitrate, copper chloride hydrate, copper chloride, copper bromide, etc.; preferably, is copper acetate monohydrate.

Said alkali is any one or more of sodium acetate, sodium carbonate, sodium bicarbonate, potassium acetate, potassium carbonate, potassium bicarbonate, cesium carbonate, lithium carbonate, magnesium acetate, calcium acetate, etc.; preferably, is sodium acetate.

The molar ratio of said rhodium catalyst, copper catalyst, alkali, compound **S3,** allenol **5** is (0.005~0.10):(0.005~1.20):(0~0.60):(1.0~3.0):1.0; preferably, is (0.010-0.025):(0.05-0.10):(0.20-0.30):(1.2-2.0):1.0.

The total synthesis method of the present invention comprises the reaction shown in the reaction formula (e): 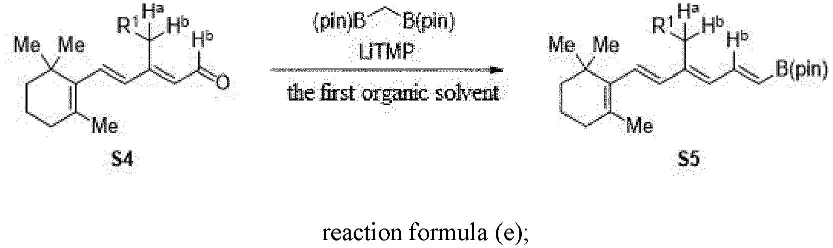 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
preferably, said R¹ is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group;
in said reaction formula (e), bis[(pinacol)boronyl]methane reacts with compound **S4** under the action of the alkali lithium 2,2,6,6-tetramethylpiperidide (LiTMP) in the first organic solvent to produce compound **S5;** among them, lithium 2,2,6,6-tetramethylpiperidide can be prepared on-site by using 2,2,6,6-tetramethylpiperidine and N-butyllithium;
said reaction shown in formula (e) specifically comprises the following steps:
   (1) protecting the reaction system with inert gas; adding 2,2,6,6-tetramethylpiperidine and the first organic solvent into a dry reaction flask, and adding n-butyllithium reagent at -90~30 °C, then continuing stirring for 15 minutes~3 hours at -90~30 °C, preparing lithium 2,2,6,6-tetramethylpiperidide solution on site; preferably, the reaction temperature is -80~0 °C, and the stirring time is 0.5∼2 hours;
   (2) dissolving bis[(pinacol)boronyl]methane in the first organic solvent, which is added to the lithium 2,2,6,6-tetramethylpiperidide solution at -90~30 °C, and then continuing stirring at -90~30 °C for 1 minute to 2 hours; preferably, adding to the lithium 2,2,6,6-tetramethylpiperidide solution at -20~20 °C, and then continuing stirring for 1~30 minutes at -20~20 °C;
   (3) dissolving compound **S4** in the first organic solvent, which is added to the mixture generated in step (2) at -90~30 °C, and then stirring at -90~30 °C for 0.5∼12 hours; preferably, adding to the mixture generated in step (2) at -80-0 °C, and then stirring the reaction at -80-0 °C for 0.5∼6 hours;
   (4) after the completion of the reaction in step (3), quenching the reaction, and obtaing compound **S5** through extraction, concentration and separation;
said first organic solvent is one or more of tetrahydrofuran, 1,4-dioxane, ether, anisole, methyl tert-butyl ether, ethylene glycol dimethyl ether, ethylene glycol diethyl ether, and toluene; preferably, is tetrahydrofuran.

The molar ratio of said **S4,** lithium 2,2,6,6-tetramethylpiperidide and bis[(pinacol)boryl]methane is 1:(1.0~2.0):(1.0~2.0), preferably is 1:(1.1-1.6):(1.1-1.6); if lithium 2,2,6,6-tetramethylpiperidide solution is prepared on site, the molar ratio of said **S4,** 2,2,6,6-tetramethylpiperidine, n-butyllithium and bis[(pinacol)boryl]methane is 1:(1.0~2.0):(1.0~2.0): (1.0~2.0), preferably, is 1:(1.1~1.6):(1.1~1.6):(1.1-1.6).

The total synthesis method of the present invention comprises the reaction shown in the reaction formula (f): 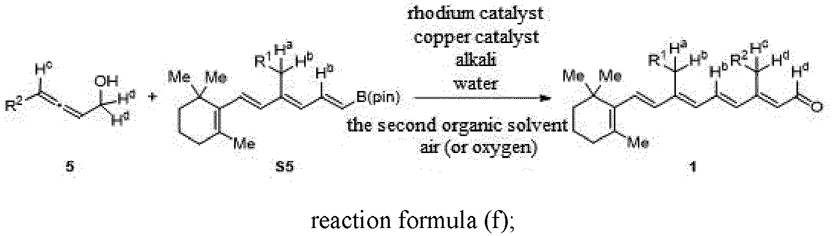 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
preferably,
said R¹ is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group;
said R² is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group.

In said reaction formula (f), allenol **5** and compound **S5** under the action of rhodium catalyst, copper catalyst, alkali, water, air or oxygen in the second organic solvent are reacted to produce compound **1;**
said reaction shown in the reaction formula (f) specifically comprises the following steps:
   (1) putting rhodium catalyst, alkali, copper catalyst, compound **S5,** allenol **5,** water and the second organic solvent into a dry reaction flask, after plugging the reaction flask with a rubber plug, inserting an air balloon or an oxygen balloon to make the reaction flask in an atmosphere of air or oxygen, and stirring the reaction at -20~60 °C for 4-96 hours;
   (2) after the completion of the reaction in step (1), filtering the mixture in the reaction flask with a short column of silica gel (or diatomaceous earth), washing with the third organic solvent, concentrating and separating to obtain compound **1;**
said second organic solvent is one or more of tetrahydrofuran, 1,4-dioxane, toluene, acetonitrile, methanol, ethanol, dichloromethane, ether, anisole, methyl tert-butyl ether, ethylene glycol dimethyl ether, ethylene glycol diethyl ether; preferably, is tetrahydrofuran.

The amount of said second organic solvent refers to the amount of allenol 5 shown in the reaction formula (f) as the basis, the amount of said second organic solvent is 1.0-20.0 mL/mmol; preferably, is 5.0 -10.0 mL/mmol.

Said third organic solvent is one or more of ethyl acetate, ether, methanol, ethanol, dichloromethane, tetrahydrofuran, 1,4-dioxane, acetone, acetonitrile, etc., preferably, is ethyl acetate; the amount of said third organic solvent refers to the amount of allenol 5 shown in the reaction formula (d) as the basis, the amount of said third organic solvent is 1.0~200 mL/mmol, preferably, is 20-100 mL/mmol.

Said rhodium catalyst is any one or more of dichloro(pentamethylcyclopentadienyl)rhodium(III) dimer, tris(acetonitrile)(pentamethylcyclopentadienyl)rhodium(III) bis(hexafluoroantimonate), rhodium(III) acetylacetonate, pentaamminechlororhodium(III) dichloride, trichlorotris(ethylenediamine)rhodium(III), potassium pentachlororhodate(III), sodium hexachlororhodate(III), potassium hexachlororhodate(III), rhodium(III) trichloride, rhodium(III) bromide, rhodium(III) iodide, rhodium(III) sulfate, rhodium(III) nitrate, potassium hexanitrorhodate(III); preferably, is dichloro(pentamethylcyclopentadienyl)rhodium(III) dimer.

Said copper catalyst is any one or more of copper acetate hydrate, copper acetate, copper sulfate hydrate, copper sulfate, copper nitrate hydrate, copper nitrate, copper chloride hydrate, copper chloride, copper bromide, etc.; preferably, is copper acetate monohydrate.

Said alkali is any one or more of sodium acetate, sodium carbonate, sodium bicarbonate, potassium acetate, potassium carbonate, potassium bicarbonate, cesium carbonate, lithium carbonate, magnesium acetate, calcium acetate, etc.; preferably, is sodium acetate.

The molar ratio of said rhodium catalyst, copper catalyst, alkali, compound **S5,** allenol **5,** and water is (0.005∼0.10): (0.005∼1.20): (0∼0.60): (1.0∼3.0): 1.0: (0∼20.0); preferably, is (0.010-0.025):(0.05-0.10):(0.20-0.30):(1.2-2.0):1.0:(2.0-5.0).

The total synthesis method of the present invention comprises the reaction shown in reaction formula (g): 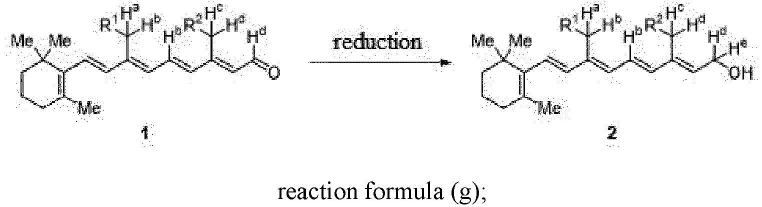 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
H^{e} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
preferably,
said R¹ is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group;
said R² is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group.

In said reaction formula (g), compound **1** is subjected to a reduction reaction to produce compound **2.**

The present invention can use any reduction reaction to produce compound **2;** preferably, compound **1** is subjected to a reduction reaction at -20~40 °C under the action of sodium borohydride with methanol or ethanol as a solvent to produce compound **2.**

The total synthesis method of the present invention comprises the reaction shown in the reaction formula (h): 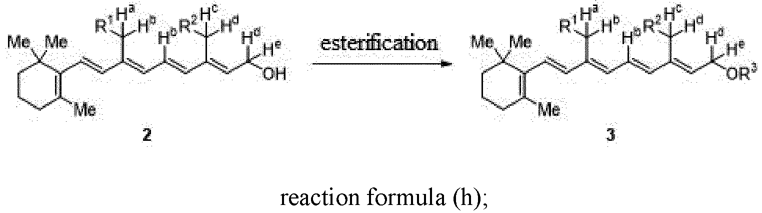 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
H^{e} is hydrogen, deuterium, tritium;
R³ is an acyl group;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
preferably,
said R¹ is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group;
said R² is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group.

In said reaction formula (h), compound **2** is esterified to produce compound **3.**

The present invention can use any esterification reaction to produce compound **3;** preferably, compound **2** and acetic anhydride are subjected to an esterification reaction at 0~60 °C under the action of triethylamine and DMAP (4-dimethylaminopyridine) with dichloromethane as a solvent to produce compound **3.**

The total synthesis method of the present invention comprises the reaction shown in the reaction formula (i): 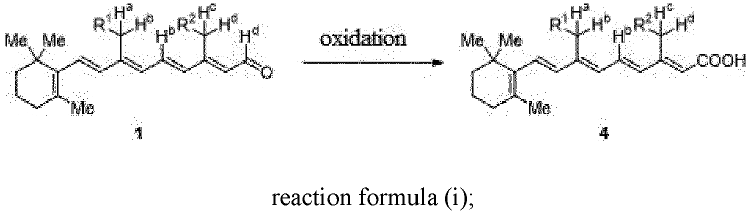 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
preferably,
said R¹ is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group;
said R² is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group.

In said reaction formula (i), compound **1** is oxidized to produce compound **4.**

The present invention can use any oxidation reaction to produce compound **4.**

The present invention also provides the application of the above-mentioned preparation method in the preparation of vitamin A and derivative thereof and deuterated compound thereof.

The present invention also provides the application of the above-mentioned preparation method in the synthesis and transformation of retinoid fragment.

The present invention also provides vitamin A and derivative thereof and deuterated compound thereof, and said molecular formula of the compounds is as follows: 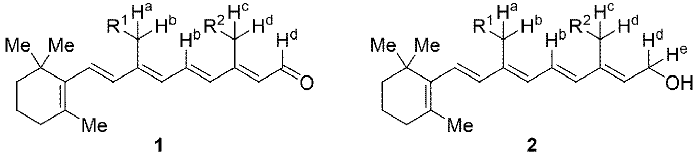 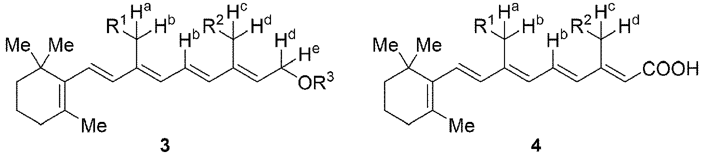 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
H^{e} is hydrogen, deuterium, tritium;
R³ is an acyl group;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
preferably,
said R¹ is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group;
said R² is hydrogen, deuterium, tritium, phenyl, aryl, heterocyclic group, C1-C40 hydrocarbyl group, C1-C40 hydrocarbyl group with functional group; said aryl group is a phenyl group with substituent at the ortho, meta and para positions, said substituent includes C1-C40 alkyl, C2-C40 alkenyl, phenyl, halogen, trifluoromethyl, C1-C40 alkoxy, C1-C40 alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur etc., which is an aliphatic ring, an aromatic ring, or a ring formed by combining two or more simple rings; in said C1-C40 hydrocarbyl group with functional group, said functional group includes acyl, hydroxyl, halogen, C1-C40 alkoxy, C1-C40 alkoxycarbonyl, formyl, acyloxy, amido, sulfonyl, sulfonyloxy, nitro, carboxy, cyano group.

The beneficial effects of the present invention include: said total synthesis method of the present invention modularly realizes the simple synthesis of vitamin A and derivative thereof with β-cyclocitral as the starting material through the repeated combination of unit reactions. Compared with the existing synthetic route, the route is concise and efficient, the steps are simple, and has the potential for industrial application. Among them, the reaction of alkenyl boron reagent and allenol is a key step, which occurs 1,4-hydrogen (or hydrogen isotope) transfer, reflecting site specificity, overcoming the difficulty of the controllable introduction of deuterium atom in the synthesis of deuterated compounds, and finally obtaining vitamin A and derivative thereof labeled with multiple hydrogen isotopes that are difficult to synthesize in the prior art.

Compared with the prior art, the total synthesis method provided by the present invention has the advantages of short steps, modularization, simple operation, flexibly controllable deuterium structure, specific stereoselectivity, and readily available raw materials and reagents; the present invention solves the problem of difficult synthesis of deuterated vitamin A and derivative thereof; the modular synthesis operation provided by the present invention can be applied to the synthesis and modification of retinoid fragment, thereby providing a powerful synthetic method for drug modification and research.

### PREFERRED EMBODIMENTS OF THE INVENTION

The following examples are given to further illustrate the specific solutions of the present invention. The process, conditions, experimental methods, and so on for implementing the present invention are all general knowledge and common knowledge in the field except for the contents specifically mentioned below, and the present invention has no special limitation.

In the reaction formula of the following examples, "equiv" refers to equivalent; "mol" refers to mole; "mmol" refers to millimoles; "mol%" refers to molar ratio, calculated on the basis of 1 equivalent of reactants; "L" refers to liter; "mL" refers to milliliters; "M" refers to mol/ L; "g" refers to gram; "mg" refers to milligram; "min" refers to minute; "h" refers to hour; "rt" refers to room temperature; "LiTMP" refers to lithium 2,2,6,6-tetramethylpiperidide; "THF" refers to tetrahydrofuran; "[Cp*RhCl₂]₂" refers to dichloro(pentamethylcyclopentadienyl)rhodium(III) dimer; "NaOAc" refers to sodium acetate; "Cu(OAc)₂·H₂O" refers to copper acetate monohydrate; "air" refers to the reaction carried out in air atmosphere; "air balloon" refers to the reaction carried out in an air atmosphere by inserting an air balloon; "Ar" refers to that the reaction carried out in an argon atmosphere; "NaIO₄" refers to sodium periodate; "NH₄OAc" refers to ammonium acetate; "MeOH" refers to methanol; "NaBH₄" refers to sodium borohydride; "DCM" refers to dichloromethane; "Ac₂O" refers to acetic anhydride; "Et₃N" refers to triethylamine; "DMAP" refers to 4-dimethylaminopyridine; "NaClO₂" refers to sodium chlorite; "NaH₂PO₄" refers to dihydrogen phosphate sodium; "t-BuOH" refers to tert-butanol; the boiling range of petroleum ether is 60-90 °C; silica gel uses 300-400 silicone; the nuclear magnetic yield is determined by ¹H NMR, and the internal standard is dibromomethane; the deuteration rate is determined by ¹H NMR.

### Example 1

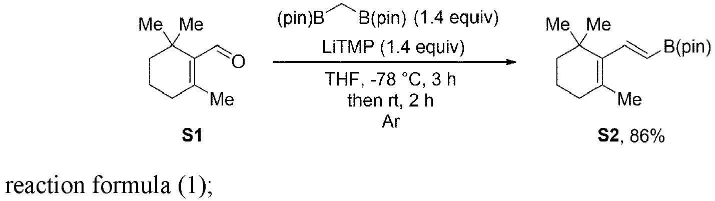

Under the protection of an argon atmosphere, 2,2,6,6-tetramethylpiperidine (11.8 mL, density 0.837 g/mL, 9.8766 g, 70 mmol) and THF (70 mL) were added to a dry reaction flask. The reaction flask was cooled to -78 °C in a dry ice acetone bath, and n-butyllithium (2.5M in hexane, 28.0mL, 70mmol) was added dropwise, and after the dropwise addition, continued stirring at -78 °C for 1 hour, and then stirred at 0 °C by an ice-water bath for 30 minutes (to produce a solution of lithium 2,2,6,6-tetramethylpiperidide). A solution of bis[(pinacol)boryl]methane (19.1414 g, 70 mmol) in THF (140 mL) was added to the reaction flask at 0 °C and continued stirring at 0 °C for 30 minutes. The reaction flask was cooled to -78 °C in a dry ice acetone bath, to which a solution of β-cyclocitral (**S1**, 8.0101g, purity 95%, 50 mmol) in THF (50 mL) was added. The reaction solution was stirred at -78 °C for 3 hours and then stirred at room temperature for 2 hours. The reaction was quenched by saturated ammonium chloride (50 mL), added with water (400 mL), and extracted with ethyl acetate (3 × 300 mL). The organic phases were combined, washed once with saturated sodium chloride solution, dried over anhydrous sodium sulfate, filtered, and rotary evaporated to remove the solvent. Silica gel column chromatography was used for separation and purification (eluent: petroleum ether, then petroleum ether/ethyl acetate=30/1) to afford a product **S2** (12.4276 g, 86%): pale yellow liquid;
**¹H NMR** (400 MHz, CDCl₃): δ = 7.01 (d, *J* = 18.4 Hz, 1 H, =CH), 5.42 (d, *J* = 18.4 Hz, 1 H, =CH), 2.00 (t, *J* = 6.2 Hz, 2 H, CH₂), 1.71 (s, 3 H, CH₃), 1.63-1.55 (m, 2 H, CH₂), 1.47-1.41 (m, 2 H, CH₂), 1.29 (s, 12 H, 4 × CH₃), 1.04 (s, 6 H, 2 × CH₃); ¹³**C NMR** (100 MHz, CDCl₃): δ = 149.5, 139.2, 130.9, 82.9, 39.8, 33.7, 33.1, 28.8, 24.8, 21.6, 19.1; **IR** (neat): v = 2977, 2928, 2866, 2827, 1615, 1459, 1379, 1370, 1344, 1317, 1266, 1212, 1164, 1144, 1109, 1028 cm⁻¹; **MS** (70 eV, EI) *m*/*z* (%): 276 (M⁺, 32.02), 161 (100).

### Example 2

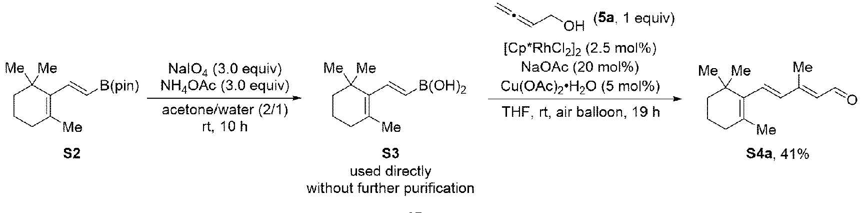

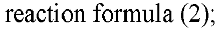

Compound **S2** was dissolved in a mixed solution of acetone and water (100 mL, 2:1), and added with sodium periodate (12.8357 g, 60 mmol) and ammonium acetate (4.6306 g, 60 mmol). The mixture was stirred at room temperature for 10 hours. Water (200 mL) was added, the mixture was extracted with ethyl acetate (4 × 100 mL), the organic phases were combined, washed with saturated sodium chloride solution, dried over anhydrous sodium sulfate, filtered and concentrated to afford a crude product **S3. S3** was used directly in the next step without purification.

[Cp*RhCl₂]₂ (201.1 mg, 0.325 mmol), NaOAc (213.5 mg, 2.6 mmol), Cu(OAc)₂·H₂O (130.1 mg, 0.65 mmol), **S3** (19.5 mmol, from previous step), THF (50 mL), **5a** (911.4 mg, 13 mmol), THF (15 mL) were added in sequence to a dry reaction tube. The reaction tube was plugged with a rubber stopper, inserted with an air balloon to keep the reaction system in an air atmosphere, and stirred at room temperature for 19 hours. The reaction solution was filtered with a short column of silica gel, washed with ethyl acetate (300 mL), and rotary evaporated to remove the solvent. Silica gel column chromatography was used for separation and purification (eluent: petroleum ether, then petroleum ether/ethyl acetate=40/1) to afford a product **S4a** (1.1517 g, 41%): yellow liquid;
**¹H NMR** (400 MHz, CDCl₃): δ = 10.13 (d, *J* = 8.4 Hz, 1 H, CHO), 6.74 (d, *J* = 16.0 Hz, 1 H, =CH), 6.21 (d, *J* = 16.0 Hz, 1 H, =CH), 5.94 (d, *J* = 8.4 Hz, 1 H, =CH), 2.31 (s, 3 H, CH₃), 2.05 (t, *J* = 6.4 Hz, 2 H, CH₂), 1.73 (s, 3 H, CH₃), 1.69-1.59 (m, 2 H, CH₂), 1.51-1.45 (m, 2 H, CH₂), 1.05 (s, 6 H, 2 × CH₃); **¹³C NMR** (100 MHz, CDCl₃): δ = 191.3, 155.0, 137.0, 135.7, 135.5, 132.7, 128.7, 39.5, 34.2, 33.2, 28.9, 21.7, 19.0, 12.9; **IR** (neat): v = 2956, 2927, 2864, 2769, 2722, 1662, 1606, 1594, 1445, 1384, 1361, 1333, 1259, 1204, 1146, 1125, 1106, 1045, 1029 cm⁻¹; **MS** (70 eV, EI) *m*/*z* (%): 218 (M⁺, 30.48), 119 (100).

### Example 3

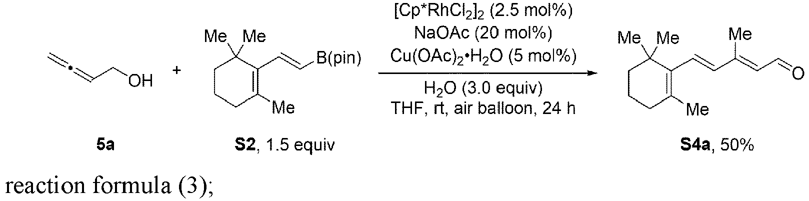

[Cp*RhCl₂]₂ (7.7 mg, 0.0125 mmol), NaOAc (8.2 mg, 0.1 mmol), Cu(OAc)₂·H₂O (5.0 mg, 0.025 mmol), **S2** (206.7 mg, 0.75 mmol), THF (1.0 mL), **5a** (35.1 mg, 0.5 mmol), THF (1.5 mL), H₂O (27 µL, 27.0 mg, 1.5 mmol) were added in sequence to a dry reaction tube. The reaction tube was plugged with a rubber stopper, inserted with an air balloon to keep the reaction system in an air atmosphere, and stirred at room temperature for 24 hours. The reaction solution was filtered with a short column of silica gel, washed with ethyl acetate (20 mL), and rotary evaporated to remove the solvent. Silica gel column chromatography was used for separation and purification (eluent: petroleum ether, then petroleum ether/ethyl acetate=50/1) to afford a product **S4a** (54.4 mg, 50%): yellow liquid;
**¹H NMR** (400 MHz, CDCl₃): δ = 10.13 (d, *J* = 8.0 Hz, 1 H, CHO), 6.74 (d, *J* = 16.0 Hz, 1 H, =CH), 6.21 (d, *J* = 16.4 Hz, 1 H, =CH), 5.94 (d, *J* = 8.0 Hz, 1 H, =CH), 2.32 (s, 3 H, CH₃), 2.05 (t, *J* = 6.2 Hz, 2 H, CH₂), 1.73 (s, 3 H, CH₃), 1.68-1.59 (m, 2 H, CH₂), 1.52-1.45 (m, 2 H, CH₂), 1.05 (s, 6 H, 2 × CH₃); **¹³C NMR** (100 MHz, CDCl₃): δ = 191.3, 155.0, 137.0, 135.7, 135.5, 132.7, 128.7, 39.5, 34.2, 33.2, 28.9, 21.7, 19.0, 12.9; **IR** (neat): v = 2956, 2928, 2864, 2769, 2723, 1661, 1606, 1594, 1445, 1384, 1361, 1333, 1259, 1204, 1146, 1125, 1106, 1045, 1029 cm⁻¹; **MS** (70 eV, EI) *m*/*z* (%): 218 (M⁺, 28.07), 119 (100).

### Example 4

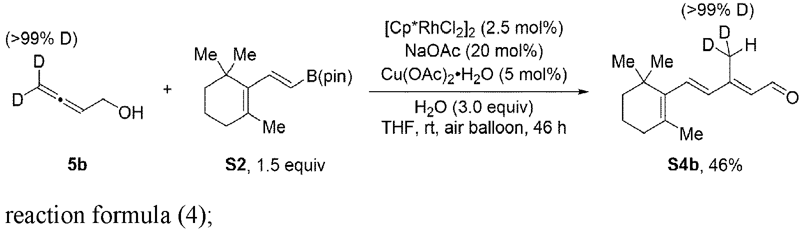

Operations were conducted by referring to Example 3. [Cp*RhCl₂]₂ (7.7 mg, 0.0125 mmol), NaOAc (8.2 mg, 0.1 mmol), Cu(OAc)₂·H₂O (5.0 mg, 0.025 mmol), **S2** (207.4 mg, 0.75 mmol), THF (1.0 mL), **5b** (36.0 mg, 0.5 mmol), THF (1.5 mL), H₂O (27 µL, 27.0 mg, 1.5 mmol) were reacted for 46 hours to afford a product **S4b** (50.5 mg, 46%) (eluting: petroleum ether/ethyl acetate=40/1): yellow liquid;
**¹H NMR** (400 MHz, CDCl₃): δ = 10.13 (d, *J* = 8.0 Hz, 1 H, CHO), 6.74 (d, *J* = 16.0 Hz, 1 H, =CH), 6.21 (d, *J* = 16.4 Hz, 1 H, =CH), 5.94 (d, *J* = 8.0 Hz, 1 H, =CH), 2.28 (s, 1 H, CD₂H), 2.05 (t, *J* = 6.2 Hz, 2 H, CH₂), 1.73 (s, 3 H, CH₃), 1.68-1.59 (m, 2 H, CH₂), 1.52-1.45 (m, 2 H, CH₂), 1.05 (s, 6 H, 2 × CH₃); **¹³C NMR** (100 MHz, CDCl₃): δ = 191.3, 155.0, 137.0, 135.6, 135.5, 132.7, 128.7, 39.5, 34.2, 33.2, 28.9, 21.7, 19.0, 12.4 (quint, *J* = 20.3 Hz); **IR** (neat): v = 2959, 2928, 2864, 2774, 2735, 1661, 1605, 1585, 1456, 1360, 1258, 1194, 1146, 1125, 1101, 1043, 1026 cm⁻¹; **MS** (70 eV, EI) *m*/*z* (%): 220 (M⁺, 26.80), 121 (100); **HRMS** calcd *m*/*z* for C₁₅H₂₀D₂O [M⁺]: 220.1791, found 220.1790.

### Example 5

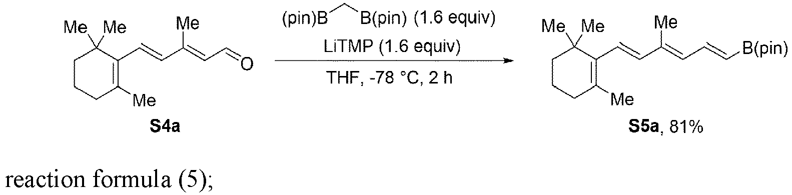

Under the protection of an argon atmosphere, 2,2,6,6-tetramethylpiperidine (905.0 mg, 6.4 mmol) and THF (6.4 mL) were added to a dry reaction flask. The reaction flask was cooled to -78 °C in a dry ice acetone bath, and added with n-butyllithium (2.5M in hexane, 2.56mL, 6.4mmol) dropwise, and after the dropwise addition, continued stirring at -78 °C for 1 hour, and then stirred at 0 °C by an ice-water bath for 30 minutes (to produce a solution of lithium 2,2,6,6-tetramethylpiperidide). A solution of bis[(pinacol)boronyl]methane (1.7170 g, 6.4 mmol) in THF (12.8 mL) was added to the reaction flask at 0 °C and continued stirring at 0 °C for 30 minutes. The reaction flask was cooled to -78 °C in a dry ice acetone bath, to which a solution of **S4a** (873.5 mg, 4 mmol) in THF (4 mL) was added. The reaction solution was stirred at -78 °C for 2 hours. The reaction was quenched by saturated ammonium chloride solution (10 mL), added with water (20 mL), and extracted with ethyl acetate (4 × 20 mL). The organic phases were combined, washed once with saturated sodium chloride solution, dried over anhydrous sodium sulfate, filtered, and rotary evaporated to remove the solvent. Silica gel column chromatography was used for separation and purification (eluent: petroleum ether, then petroleum ether/ethyl acetate=50/1) to afford a product **S5a** (1.1036 g, 81%): yellow liquid;
**¹H NMR** (400 MHz, CDCl₃): δ = 7.41 (dd, *J*₁ = 17.4 Hz, *J*₂ = 11.4 Hz, 1 H, =CH), 6.26 (d, *J* = 16.0 Hz, 1 H, =CH), 6.31-6.22 (m, 2 H, 2 x =CH), 5.56 (d, *J* = 17.2 Hz, 1 H, =CH), 2.08-1.97 (m, 5 H, CH₂ and CH₃), 1.70 (s, 3 H, CH₃), 1.67-1.58 (m, 2 H, CH₂), 1.49-1.43 (m, 2 H, CH₂), 1.28 (s, 12 H, 4 × CH₃), 1.02 (s, 6 H, 2 × CH₃); ¹³C **NMR** (100 MHz, CDCl₃): δ =145.7, 139.6, 137.6, 137.3, 131.7, 129.8, 128.7, 83.1, 39.6, 34.2, 33.1, 28.9, 24.7, 21.7, 19.2, 12.9; **IR** (neat): v = 3431, 2976, 2928, 2865, 1718, 1669, 1616, 1601, 1576, 1472, 1456, 1379, 1362, 1338, 1271, 1142, 1105 cm⁻¹; **MS** (70 eV, EI) *m*/*z* (%): 342 (M⁺, 12.16), 129 (100).

### Example 6

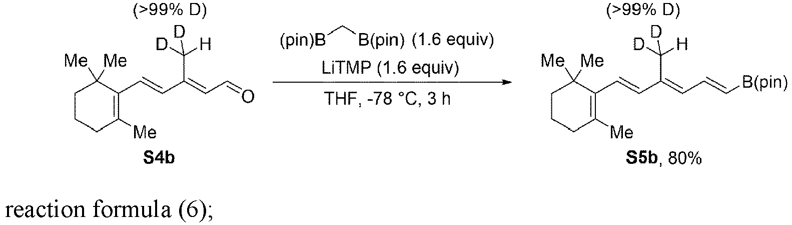

Under the protection of an argon atmosphere, 2,2,6,6-tetramethylpiperidine (0.81 mL, d=0.837 g/mL, 678.0 mg, 4.8 mmol) and THF (4.8 mL) were added to a dry reaction flask. The reaction flask was cooled to -78 °C in a dry ice acetone bath, and added with n-butyllithium (2.5M in hexane, 1.92mL, 4.8mmol) dropwise, and after the dropwise addition, continued stirring at -78 °C for 1 hour, and then stirred at 0 °C by an ice-water bath for 30 minutes (to produce a solution of lithium 2,2,6,6-tetramethylpiperidide). A solution of bis[(pinacol)boryl]methane (1.2894 g, 4.8 mmol) in THF (9.6 mL) was added to the reaction flask at 0 °C and continued stirring at 0 °C for 30 minutes. The reaction flask was cooled to -78 °C in a dry ice acetone bath, to which a solution of **S4b** (661.1 mg, 3.0 mmol) in THF (3 mL) was added. The reaction solution was stirred at -78 °C for 3 hours. The reaction was quenched by saturated ammonium chloride solution (3 mL), added with water (30 mL), and extracted with ethyl acetate (4 × 30 mL). The organic phases were combined, washed once with saturated sodium chloride solution, dried over anhydrous sodium sulfate, filtered, and rotary evaporated to remove the solvent. Silica gel column chromatography was used for separation and purification (eluent: petroleum ether, then petroleum ether/ethyl acetate=40/1) to afford a product **S5b** (823.8 mg, 80%): yellow liquid;
**¹H NMR** (400 MHz, CDCl₃): δ = 7.41 (dd, *J*₁ = 17.2 Hz, *J*₂ = 11.2 Hz, 1 H, =CH), 6.26 (d, *J* = 16.0 Hz, 1 H, =CH), 6.17-6.05 (m, 2 H, 2 x =CH), 5.58 (d, *J* = 17.2 Hz, 1 H, =CH), 2.01 (t, *J* = 6.0 Hz, 2 H, CH₂), 1.96 (s, 1 H, CD₂H), 1.70 (s, 3 H, CH₃), 1.65-1.57 (m, 2 H, CH₂), 1.48-1.43 (m, 2 H, CH₂), 1.28 (s, 12 H, 4 × CH₃), 1.02 (s, 6 H, 2 × CH₃); **¹³C NMR** (100 MHz, CDCl₃): δ =145.7, 139.6, 137.6, 137.3, 131.7, 129.8, 128.7, 83.1, 39.6, 34.2, 33.1, 28.9, 24.7, 21.7, 19.2, 12.4 (quint, *J* = 19.4 Hz); **IR** (neat): v = 2976, 2928, 2864, 1614, 1597, 1572, 1456, 1379, 1371, 1341, 1317, 1269, 1142, 1103 cm⁻¹; **MS** (70 eV, EI) *m*/*z* (%): 344 (M⁺, 90.1), 101 (100); **HRMS** calcd *m*/*z* for C₂₂H₃₃D₂¹¹BO₂ [M⁺]: 344.2850, found 344.2853.

### Example 7

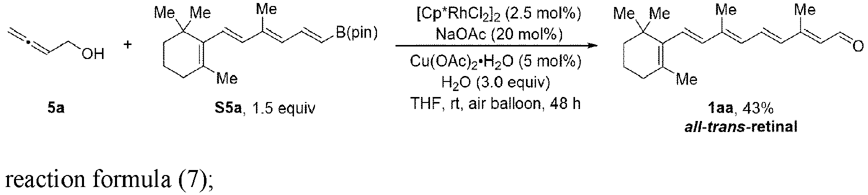

[Cp*RhCl₂]₂ (3.1 mg, 0.005 mmol), NaOAc (3.3 mg, 0.04 mmol), Cu(OAc)₂·H₂O (2.0 mg, 0.01 mmol), **S5a** (THF solution, 0.5M, 600 µL, 0.3 mmol), **5a** (14.1 mg, 0.2 mmol), THF (1 mL), H₂O (11 µL, 11.0 mg, 0.6 mmol) were added in sequence to a dry reaction tube. The reaction tube was plugged with a rubber stopper, inserted with an air balloon to keep the reaction system in an air atmosphere, and stirred at room temperature for 48 hours in the dark. The reaction solution was filtered with a short column of diatomaceous earth, washed with ethyl acetate (30 mL), and rotary evaporated to remove the solvent. The silica gel preparation plate was used for separation and purification (the silica gel preparation plate was alkalized with petroleum ether containing 5% triethylamine by volume) (eluent: petroleum ether/ethyl acetate=20/1) to afford a product retinal (**1aa**, 24.6 mg, 43%): orange solid; melting point 59.5-60.8 °C (petroleum ether recrystallization ) (reported in literature (Ball, S.; Goodwin, T. W.; Morton, R. A. Biochem. J. 1948, 42, 516): 61-62 °C);
**¹H NMR** (400 MHz, CDCl₃): δ = 10.11 (d, *J* = 8.0 Hz, 1 H, CHO), 7.14 (dd, *J*₁ = 15.2 Hz, *J*₂ = 11.6 Hz, 1 H, =CH), 6.43-6.29 (m, 2 H, 2 x =CH), 6.22-6.13 (m, 2 H, 2 x =CH), 5.97 (d, *J=* 8.0 Hz, 1 H, =CH), 2.33 (d, *J=* 0.8 Hz, 3 H, CH₃), 2.08-2.00 (m, 5 H, CH₃ and CH₂), 1.72 (s, 3 H, CH₃), 1.67-1.58 (m, 2 H, CH₂), 1.51-1.44 (m, 2 H, CH₂), 1.04 (s, 6 H, 2 × CH₃); **¹³C NMR** (100 MHz, CDCl₃): δ = 191.1, 154.8, 141.3, 137.6, 137.1, 134.5, 132.5, 130.5, 129.7, 129.4, 129.0, 39.6, 34.2, 33.1, 28.9, 21.7, 19.2, 13.1, 13.0; **IR** (neat): v = 2956, 2927, 2864, 2826, 2092, 1651, 1568, 1457, 1447, 1353, 1195, 1169, 1044 cm⁻¹; **MS** (70 eV, EI) *m*/*z* (%): 284 (M⁺, 82.64), 128 (100).

### Example 8

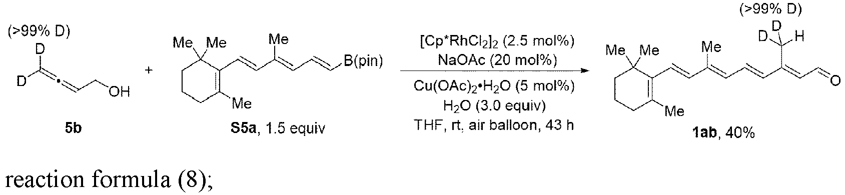

Operations were conducted by referring to Example 7. [Cp*RhCl₂]₂ (3.2 mg, 0.005 mmol), NaOAc (3.3 mg, 0.04 mmol), Cu(OAc)₂·H₂O (2.0 mg, 0.01 mmol), **S5a** (THF solution, 0.5 M, 600 µL, 0.3 mmol), **5b** (14.4 mg, 0.2 mmol), THF (1 mL), H₂O (11 µL, 11.0 mg, 0.6 mmol) were reacted for 43 hours to afford a product **1ab** (22.9 mg, 40%) (silica gel preparation plate was alkalized with petroleum ether containing 5% triethylamine by volume) (eluent: petroleum ether/ethyl acetate=20/1): orange liquid;
**¹H NMR** (400 MHz, CDCl₃): δ = 10.10 (d, *J=* 8.0 Hz, 1 H, CHO), 7.14 (dd, *J*₁ = 14.8 Hz, *J*₂ = 11.6 Hz, 1 H, =CH), 6.43-6.29 (m, 2 H, 2 x =CH), 6.22-6.11 (m, 2 H, 2 x =CH), 5.97 (d, *J* = 8.0 Hz, 1 H, =CH), 2.29 (s, 1 H, CD₂H), 2.09-1.99 (m, 5 H, CH₃ and CH₂), 1.72 (s, 3 H, CH₃), 1.66-1.58 (m, 2 H, CH₂), 1.51-1.44 (m, 2 H, CH₂), 1.04 (s, 6 H, 2 × CH₃); **¹³C NMR** (100 MHz, CDCl₃): δ = 191.1, 154.8, 141.3, 137.6, 137.1, 134.5, 132.5, 130.5, 129.7, 129.4, 129.0, 39.6, 34.2, 33.1, 28.9, 21.7, 19.2, 13.0, 12.4 (quint, *J* = 19.4 Hz); **IR** (neat): v = 2956, 2925, 2861, 1655, 1569, 1447, 1375, 1353, 1265, 1194, 1161, 1107 cm⁻¹; **MS** (70 eV, EI) *m*/*z* (%): 286 (M⁺, 100); **HRMS** calcd *m*/*z* for C₂₀H₂₆D₂O [M⁺]: 286.2260, found 286.2259.

### Example 9

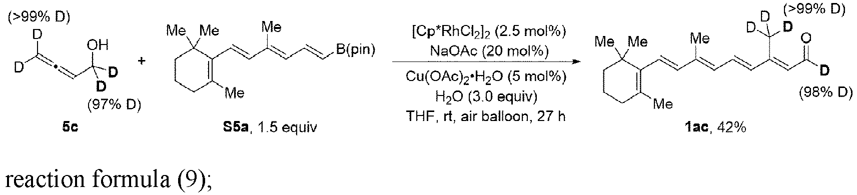

Operations were conducted by referring to Example 7. [Cp*RhCl₂]₂ (3.1 mg, 0.005 mmol), NaOAc (3.3 mg, 0.04 mmol), Cu(OAc)₂·H₂O (2.0 mg, 0.01 mmol), **S5a** (THF solution, 0.5 M, 600 µL, 0.3 mmol), **5c** (14.8 mg, 0.2 mmol), THF (1 mL), H₂O (11 µL, 11.0 mg, 0.6 mmol) were reacted for 27 hours to afford a product **1ac** (24.0 mg, 42%) (silica gel preparation plate was alkalized with petroleum ether containing 5% triethylamine by volume) (eluent: petroleum ether/ethyl acetate=40/1): orange liquid;
**¹H NMR** (400 MHz, CDCl₃): δ = 7.14 (dd, *J*₁ = 15.2 Hz, *J*₂ = 11.6 Hz, 1 H, =CH), 6.42-6.30 (m, 2 H, 2 x =CH), 6.22-6.13 (m, 2 H, 2 x =CH), 5.97 (s, 1 H, =CH), 2.08-2.00 (m, 5 H, CH₃ and CH₂), 1.72 (s, 3 H, CH₃), 1.66-1.58 (m, 2 H, CH₂), 1.51-1.44 (m, 2 H, CH₂), 1.04 (s, 6 H, 2 × CH₃); ¹³C **NMR** (100 MHz, CDCl₃): δ = 190.8 (t, *J* = 26.1 Hz), 154.8, 141.3, 137.6, 137.0, 134.5, 132.5, 130.5, 129.7, 129.4, 129.0, 39.6, 34.2, 33.1, 28.9, 21.7, 19.2, 13.0, 12.4 (hept, *J* = 19.3 Hz); **IR** (neat): v = 2956, 2927, 2864, 2826, 2092, 1651, 1568, 1457, 1447, 1353, 1195, 1169, 1044 cm⁻¹; **MS** (70 eV, EI) *mlz* (%): 288 (M⁺, 100); **HRMS** calcd *m*/*z* for C₂₀H₂₄D₄O [M⁺]: 288.2386, found 288.2387.

### Example 10

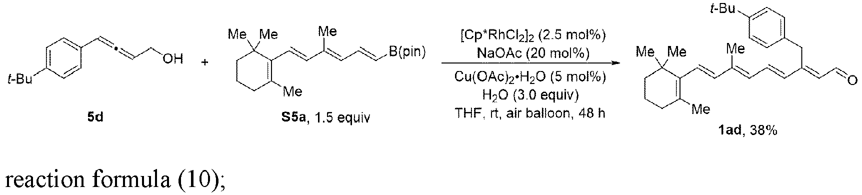

Operations were conducted by referring to Example 7. [Cp*RhCl₂]₂ (3.1 mg, 0.005 mmol), NaOAc (3.3 mg, 0.04 mmol), Cu(OAc)₂·H₂O (2.0 mg, 0.01 mmol), **S5a** (THF solution, 0.5 M, 600 µL, 0.3 mmol), **5d** (40.4 mg, 0.2 mmol), THF (1 mL), H₂O (11 µL, 11.0 mg, 0.6 mmol) were reacted for 48 hours to afford a product **1ad** (31.7 mg, 38%) (silica gel preparation plate was alkalized with petroleum ether containing 5% triethylamine by volume) (eluent: petroleum ether/ethyl acetate=20/1): orange liquid;
**¹H NMR** (400 MHz, CDCl₃): δ = 10.11 (d, *J* = 8.0 Hz, 1 H, CHO), 7.35-7.30 (m, 2 H, Ar-H), 7.21-7.12 (m, 3 H, =CH and Ar-H), 6.37-6.27 (m, 2 H, 2 x =CH), 6.17-6.08 (m, 3 H, 3 x =CH), 4.13 (s, 2 H, CH₂), 2.02 (t, *J =* 6.0 Hz, 2 H, CH₂), 1.90 (s, 3 H, CH₃), 1.69 (s, 3 H, CH₃), 1.66-1.58 (m, 2 H, CH₂), 1.49-1.42 (m, 2 H, CH₂), 1.30 (s, 9 H, 3 × CH₃), 1.01 (s, 6 H, 2 × CH₃); **¹³C NMR** (100 MHz, CDCl₃): δ = 191.4, 156.7, 149.6, 141.5, 137.6, 137.0, 135.3, 133.9, 133.2, 130.5, 129.9, 129.5, 129.2, 127.5, 125.8, 39.5, 34.4, 34.3, 33.1, 32.7, 31.3, 28.9, 21.7, 19.1, 12.9; **IR** (neat): v = 2957, 2924, 2864, 2737, 1657, 1572, 1458, 1362, 1346, 1267, 1159, 1099 cm⁻¹; **MS** (70 eV, EI) *m*/*z* (%): 416 (M⁺, 58.4), 147 (100); **HRMS** calcd *m*/*z* for C₃₀H₄₀O [M⁺]: 416.3074, found 416.3075.

### Example 11

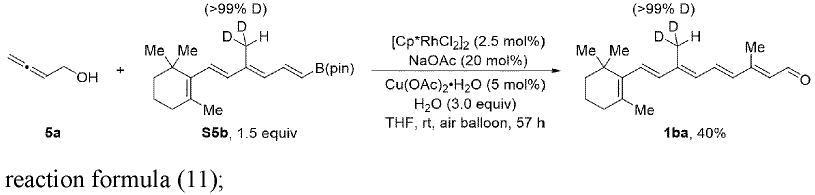

Operations were conducted by referring to Example 7. [Cp*RhCl₂]₂ (3.1 mg, 0.005 mmol), NaOAc (3.3 mg, 0.04 mmol), Cu(OAc)₂·H₂O (2.0 mg, 0.01 mmol), **SSb** (THF solution, 0.5 M, 600 µL, 0.3 mmol), **5a** (14.0 mg, 0.2 mmol), THF (1 mL), H₂O (11 µL, 11.0 mg, 0.6 mmol) were reacted for 57 hours to afford a product **1ba** (23.1 mg, 40%) (silica gel preparation plate was alkalized with petroleum ether containing 5% triethylamine by volume) (eluent: petroleum ether/ethyl acetate=20/1): orange liquid;
**¹H NMR** (400 MHz, CDCl₃): δ = 10.11 (d, *J* = 8.0 Hz, 1 H, CHO), 7.14 (dd, *J*₁ = 14.8 Hz, *J*₂ = 11.6 Hz, 1 H, =CH), 6.44-6.29 (m, 2 H, 2 x =CH), 6.22-6.10 (m, 2 H, 2 x =CH), 5.97 (d, *J* = 8.0 Hz, 1 H, =CH), 2.33 (s, 3 H, CH₃), 2.07-1.97 (m, 3 H, CD₂H and CH₂), 1.72 (s, 3 H, CH₃), 1.66-1.58 (m, 2 H, CH₂), 1.50-1.43 (m, 2 H, CH₂), 1.04 (s, 6 H, 2 × CH₃); **¹³C NMR** (100 MHz, CDCl₃): δ = 191.1, 154.8, 141.3, 137.6, 137.1, 134.5, 132.5, 130.5, 129.7, 129.4, 129.0, 39.6, 34.3, 33.1, 28.9, 21.7, 19.2, 13.1, 12.5 (quint, *J* = 19.2 Hz); **IR** (neat): v = 2959, 2924, 2862, 2762, 1655, 1574, 1447, 1385, 1159, 1132, 1109, 1043 cm⁻¹; **MS** (70 eV, EI) *m*/*z* (%): 286 (M⁺, 100); **HRMS** calcd *m*/*z* for C₂₀H₂₆D₂O [M⁺]: 286.2260, found 286.2263.

### Example 12

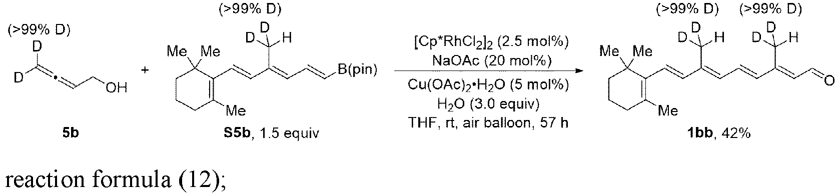

Operations were conducted by referring to Example 7. [Cp*RhCl₂]₂ (3.1 mg, 0.005 mmol), NaOAc (3.3 mg, 0.04 mmol), Cu(OAc)₂·H₂O (2.0 mg, 0.01 mmol), **SSb** (THF solution, 0.5 M, 600 µL, 0.3 mmol), **5b** (14.5 mg, 0.2 mmol), THF (1 mL), H₂O (11 µL, 11.0 mg, 0.6 mmol) were reacted for 57 hours to afford a product **1bb** (24.2 mg, 42%) (silica gel preparation plate was alkalized with petroleum ether containing 5% triethylamine by volume) (eluent: petroleum ether/ethyl acetate=20/1): orange liquid;
**¹H NMR** (400 MHz, CDCl₃): δ = 10.10 (d, *J* = 8.0 Hz, 1 H, CHO), 7.13 (dd, *J*₁ = 14.8 Hz, *J*₂ = 11.6 Hz, 1 H, =CH), 6.45-6.30 (m, 2 H, 2 x =CH), 6.22-6.12 (m, 2 H, 2 × =CH), 5.97 (d, *J* = 8.0 Hz, 1 H, =CH), 2.29 (s, 1 H, CD₂H), 2.03 (t, *J* = 6.2 Hz, 2 H, CH₂), 1.99 (s, 1 H, CD₂H), 1.72 (s, 3 H, CH₃), 1.67-1.58 (m, 2 H, CH₂), 1.51-1.44 (m, 2 H, CH₂), 1.04 (s, 6 H, 2 × CH₃); **¹³C NMR** (100 MHz, CDCl₃): δ = 191.1, 154.8, 141.2, 137.6, 137.0, 134.5, 132.5, 130.5, 129.7, 129.4, 129.0, 39.6, 34.2, 33.1, 28.9, 21.7, 19.1, 12.6 (quint, *J* = 19.6 Hz), 12.5 (quint, *J* = 19.4 Hz); **IR** (neat): v = 2955, 2926, 2862, 1655, 1570, 1456, 1356, 1269, 1186, 1159, 1105 cm⁻¹; **MS** (70 eV, EI) *m*/*z* (%): 288 (M⁺, 50.0), 93 (100); **HRMS** calcd *m*/*z* for C₂₀H₂₄D₄O [M⁺]: 288.2386, found 288.2388.

### Example 13

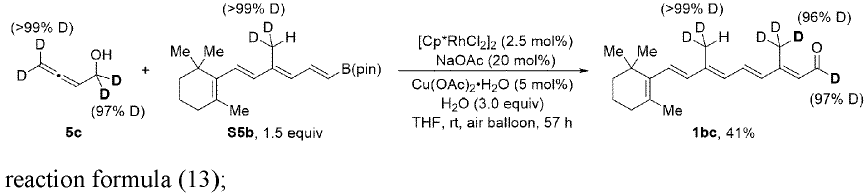

Operations were conducted by referring to Example 7. [Cp*RhCl₂]₂ (3.1 mg, 0.005 mmol), NaOAc (3.3 mg, 0.04 mmol), Cu(OAc)₂·H₂O (2.0 mg, 0.01 mmol), **SSb** (THF solution, 0.5 M, 600 µL, 0.3 mmol), **5c** (14.8 mg, 0.2 mmol), THF (1 mL), H₂O (11 µL, 11.0 mg, 0.6 mmol) were reacted for 57 hours to afford a product **1bc** (23.7 mg, 41%) (silica gel preparation plate was alkalized with petroleum ether containing 5% triethylamine by volume) (eluent: petroleum ether/ethyl acetate=20/1): orange liquid;
**¹H NMR** (400 MHz, CDCl₃): δ = 7.13 (dd, *J*₁ = 14.8 Hz, *J*₂ = 11.6 Hz, 1 H, =CH), 6.45-6.29 (m, 2 H, 2 x =CH), 6.25-6.11 (m, 2 H, 2 x =CH), 5.97 (s, 1 H, =CH), 2.08-1.96 (m, 3 H, CD₂H and CH₂), 1.72 (s, 3 H, CH₃), 1.68-1.59 (m, 2 H, CH₂), 1.51-1.44 (m, 2 H, CH₂), 1.04 (s, 6 H, 2 x CH₃); ¹³C **NMR** (100 MHz, CDCl₃): δ = 190.8 (t, *J* = 26.1 Hz), 154.8, 141.2, 137.6, 137.0, 134.5, 132.5, 130.5, 129.7, 129.4, 129.0, 39.6, 34.2, 33.1, 28.9, 21.7, 19.1, 12.5 (quint, *J* = 19.2 Hz), 12.3 (hept, *J* = 19.3 Hz); **IR** (neat): v = 2959, 2926, 2862, 2824, 2093, 1647, 1566, 1551, 1456, 1358, 1186, 1165, 1043 cm⁻¹; **MS** (70 eV, EI) *m*/*z* (%): 290 (M⁺, 100); **HRMS** calcd *m*/*z* for C₂₀H₂₂D₆O [M⁺]: 290.2511, found 290.2511.

### Example 14

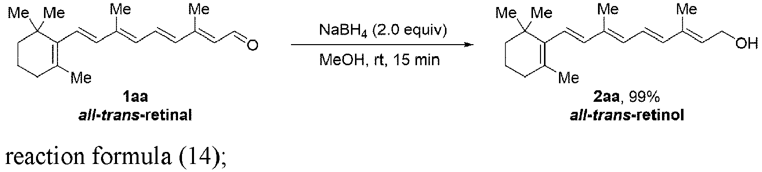

Retinal (**1aa**, 57.0 mg, 0.2 mmol) and methanol (1 mL) were added to a dry reaction tube, and sodium borohydride (15.2 mg, 0.4 mmol) was added while stirring. The mixture was stirred at room temperature for 15 minutes in the dark. The reaction was quenched with water (5 mL), and the mixture was extracted with ethyl acetate (4 × 5 mL). The organic phases were combined, washed twice with saturated sodium chloride solution, dried over anhydrous sodium sulfate, filtered, and rotary evaporated to remove the solvent to afford vitamin A (**2aa**, 57.1 mg, 99%): yellow liquid;
¹**H NMR** (400 MHz, CDCl₃): δ = 6.61 (dd, *J*₁ = 15.0 Hz, *J*₂ = 11.4 Hz, 1 H, =CH), 6.29 (d, *J* = 14.8 Hz, 1 H, =CH), 6.21-6.07 (m, 3 H, 3 x =CH), 5.69 (t, *J* = 6.8 Hz, 1 H, =CH), 4.31 (d, *J* = 7.2 Hz, 2 H, OCH₂), 2.01 (t, *J* = 6.2 Hz, 2 H, CH₂), 1.96 (s, 3 H, CH₃), 1.86 (s, 3 H, CH₃), 1.71 (s, 3 H, CH₃), 1.65-1.57 (m, 2 H, CH₂), 1.49-1.43 (m, 2 H, CH₂), 1.02 (s, 6 H, 2 × CH₃); **¹³C NMR** (100 MHz, CDCl₃): δ = 137.8, 137.6, 136.9, 136.3, 136.2, 130.05, 129.95, 129.3, 126.7, 125.2, 59.5, 39.6, 34.2, 33.0, 28.9, 21.7, 19.2, 12.7, 12.6; **IR** (neat): v = 3384, 3039, 2926, 2863, 1662, 1629, 1572, 1444, 1376, 1359, 1266, 1203, 1079, 1006 cm⁻¹; **MS** (70 eV, EI) *m*/*z* (%): 286 (M⁺, 97.12), 91 (100).

### Example 15

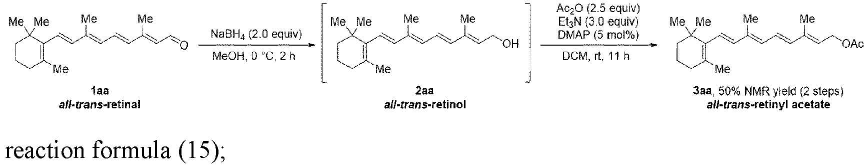

Retinal (**1aa**, 142.2 mg, 0.5 mmol) and methanol (2.5 mL) were added to a dry reaction tube, the reaction tube was placed in an ice-water bath at 0 °C, and added with sodium borohydride (38.0 mg, 1.0 mmol) while stirring. The mixture was stirred under an ice-water bath at 0 °C for 2 hours in the dark. The reaction was quenched with water (10 mL), and the mixture was extracted with ethyl acetate (4 × 10 mL). The organic phases were combined, washed once with saturated sodium chloride solution, dried over anhydrous sodium sulfate, filtered, and rotary evaporated to remove the solvent to afford a crude product: vitamin A (**2aa**).

The vitamin A (**2aa**) crude product was dissolved in dichloromethane (3 mL), and triethylamine (151.6 mg, 1.5 mmol), dichloromethane (1 mL), 4-dimethylaminopyridine ( DMAP, 3.1 mg, 0.025 mmol), acetic anhydride (127.6 mg, 1.25 mmol), dichloromethane (1 mL) were added in sequence while stirring at room temperature. The mixture was stirred at room temperature for 11 hours in the dark. The reaction was quenched with saturated sodium bicarbonate solution (10 mL), then added with dichloromethane (10 mL), washed, then separated. The organic phase was then washed once with saturated sodium bicarbonate solution (15 mL), washed once with saturated sodium chloride solution, dried over anhydrous sodium sulfate, filtered, and rotary evaporated to remove the solvent to afford a crude product: vitamin A acetate (**3aa**). The NMR yield was 50% (two steps).

### Example 16

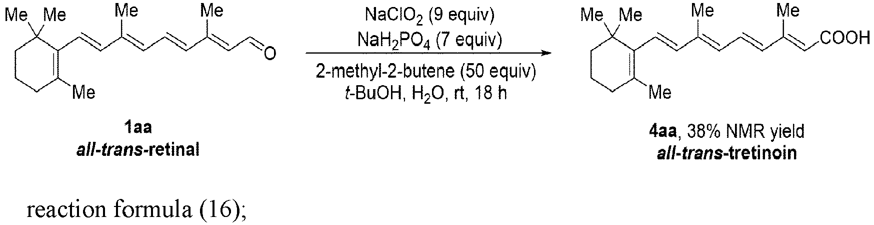

Retinal **(1aa,** 57.0 mg, 0.2 mmol), tert-butanol (4 mL), 2-methyl-2-butene (1.2 mL, density 0.662 g/mL, 794.4 mg, purity 90%, 10 mmol) were added in sequence to a dry reaction tube. NaClO₂ (163.2 mg, 1.8 mmol) and NaH₂PO₄ (167.9 mg, 1.4 mmol) were dissolved in water (2 mL) and the solution was added dropwise to the reaction mixture. The reaction mixture was stirred at room temperature for 18 hours, then rotary evaporated to remove the components with low-boiling point, added with water (10 mL), adjusted to pH to 3 with aqueous 1M hydrochloric acid solution, extracted with ether (4 × 10 mL), and washed once with saturated sodium chloride solution, dried over anhydrous sodium sulfate, filtered, and rotary evaporated to remove the solvent to afford a crude product: retinoic acid (**4aa**), NMR yield of 38%.

The protection content of the present invention is not limited to the above examples. Without departing from the spirit and scope of the inventive concept, variations and advantages that can occur to those skilled in the art are included in the present invention, and the appended claims are the scope of protection.

## Claims

1. A total synthesis method of vitamin A and derivative thereof and deuterated compound thereof, wherein, said total synthesis method comprises the following steps:
(1) the alkenyl boronate shown in **S2** is produced from β-cyclocitral shown in **S1** and bis[(pinacol)boryl]methane under the action of lithium tetramethylpiperidide in the first organic solvent;
(2) the 2-alkenal shown in **S4** is produced from the alkenyl boronate shown in **S2** generated in said step (1) and the allenol shown in **5** under the action of rhodium catalyst, copper catalyst, alkali, water, air or oxygen in the second organic solvent; or, the alkenyl boronate shown in said **S2** is first hydrolyzed into the alkenyl boronic acid shown in **S3,** and then combined with the allenol shown in **5,** under the action of rhodium catalyst, copper catalyst, alkali, air or oxygen, in the second organic solvent to produce 2-alkenal shown in **S4;**
(3) the alkenyl boronate shown in **S5** is produced from the 2-alkenal shown in **S4** generated in said step (2) and bis[(pinacol)boronyl]methane under the action of lithium tetramethylpiperidide in the first organic solvent;
(4) the retinal or the retinal with substituent shown in **1** is produced from the alkenyl boronate shown in **S5** generated in said step (3) and the allenol shown in **5,** under the action of rhodium catalyst, copper catalyst, alkali, water, air or oxygen, in the second organic solvent;
(5) the retinal or the retinal with substituent shown in 1 generated in said step (4) is subjected to a reduction reaction to produce the vitamin A or the vitamin A with substituent shown in **2;**
(6) the vitamin A or the vitamin A with substituent shown in **2** generated in said step (5) is subjected to an esterification reaction to produce the vitamin A ester or the vitamin A ester with substituent shown in **3;**
(7) the retinal or the retinal with substituent shown in **1** generated in said step (4) is subjected to an oxidation reaction to produce the vitamin A acid or the vitamin A acid with substituent shown in **4;**
in said above-mentioned step (2), and/or said step (4), when using the allenol shown in **5** labeled with hydrogen isotope, finally obtaining the vitamin A or the vitamin derivative labeled with hydrogen isotope;
the synthetic route of said total synthesis method is shown in the following formula (A): 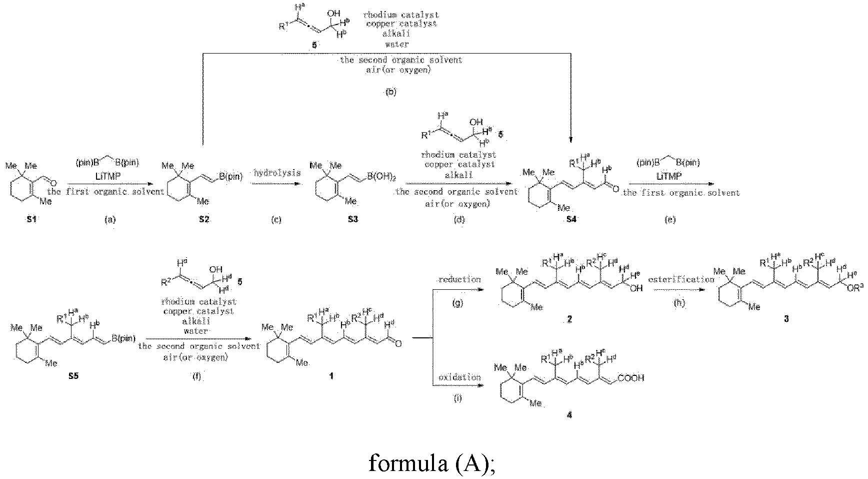
wherein,
said H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
H^{e} is hydrogen, deuterium , tritium;
R³ is an acyl group;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group.

2. The total synthesis method of claim 1, wherein, said total synthesis method comprises the reaction shown in reaction formula (a): 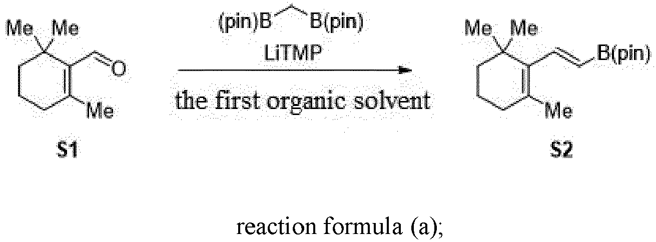
in said reaction formula (a), bis[(pinacol)boronyl]methane reacts with β-cyclocitral shown in **S1** under the action of the alkali lithium 2,2,6,6-tetramethylpiperidide LiTMP in the first organic solvent to produce compound S2; among them, said lithium 2,2,6,6-tetramethylpiperidide is prepared by using 2,2,6,6-tetramethylpiperidine and N-butyllithium;
the reaction shown in formula (a) specifically comprises the following steps:
(1) protecting the reaction system with inert gas; adding 2,2,6,6-tetramethylpiperidine and the first organic solvent into a dry reaction flask, and adding n-butyllithium reagent at -90~30 °C, then continuing stirring for 15 minutes~3 hours at -90~30 °C, preparing lithium 2,2,6,6-tetramethylpiperidide solution on site;
(2) dissolving bis[(pinacol)boronyl]methane in said first organic solvent, which is added to the lithium 2,2,6,6-tetramethylpiperidide solution generated in said step (1) at -90~30 °C, and then continuing stirring at -90~30 °C for 1 minute to 2 hours;
(3) dissolving β-cyclocitral in said first organic solvent, which is added to the mixture generated in step (2) at -90~30°C, and then stirring the reaction at -90~30 °C for 0.5∼12 hours;
(4) after the completion of the reaction in said step (3), quenching the reaction, and obtaining compound **S2** through extraction, concentration and separation;
said first organic solvent is one or more of tetrahydrofuran, 1,4-dioxane, ether, anisole, methyl tert-butyl ether, ethylene glycol dimethyl ether, ethylene glycol diethyl ether, and toluene;
the molar ratio of said β-cyclocitral shown in **S1,** lithium 2,2,6,6-tetramethylpiperidide and bis[(pinacol)boryl]methane is 1:(1.0-2.0):(1.0-2.0); if lithium 2,2,6,6-tetramethylpiperidide solution is prepared on site, the molar ratio of said β-cyclocitral shown in **S1,** 2,2,6,6-tetramethylpiperidine, n-butyllithium and bis[(pinacol)boryl]methane is 1:(1.0~2.0):(1.0~2.0 ): (1.0~2.0).

3. The total synthesis method of claim 1, wherein, said total synthesis method comprises the reaction shown in reaction formula (b): 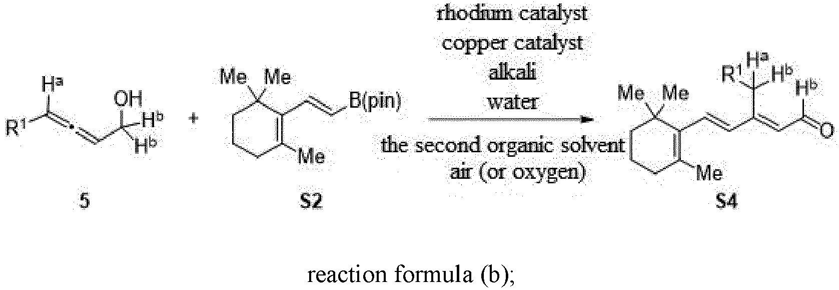 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
in said reaction formula (b), allenol **5** and compound **S2** under the action of rhodium catalyst, copper catalyst, alkali, water, air or oxygen in the second organic solvent are reacted to produce compound **S4;**
said reaction shown in the reaction formula (b) specifically comprises the following steps:
(1) putting rhodium catalyst, alkali, copper catalyst, compound **S2,** allenol **5,** water and the second organic solvent into a dry reaction flask, after plugging the reaction flask with a rubber plug, inserting an air balloon or an oxygen balloon to make the reaction flask in an atmosphere of air or oxygen, and stirring the reaction at -20~60 °C for 4∼96 hours;
(2) after the completion of the reaction in step (1), filtering the mixture in the reaction flask with a short column of silica gel or diatomaceous earth, washing with the third organic solvent, then concentrating, separating to obtain compound **S4;**
said second organic solvent is one or more of tetrahydrofuran, 1,4-dioxane, toluene, acetonitrile, methanol, ethanol, dichloromethane, ether, anisole, methyl tert-butyl ether, ethylene glycol dimethyl ether, ethylene glycol diethyl ether; the amount of said second organic solvent refers to the amount of allenol **5** shown in the reaction formula (b) as the basis, the amount of said second organic solvent is 1.0-20.0 mL/mmol;
said third organic solvent is one or more of ethyl acetate, ether, methanol, ethanol, dichloromethane, tetrahydrofuran, 1,4-dioxane, acetone, acetonitrile; the amount of said third organic solvent refers to the amount of allenol **5** shown in the reaction formula (b) as the basis, the amount of the third organic solvent is 1.0~200 mL/mmol;
said rhodium catalyst is any one or more of dichloro(pentamethylcyclopentadienyl)rhodium(III) dimer, tris(acetonitrile)(pentamethylcyclopentadienyl)rhodium(III) bis(hexafluoroantimonate), rhodium(III) acetylacetonate, pentaamminechlororhodium(III) dichloride, trichlorotris(ethylenediamine)rhodium(III), potassium pentachlororhodate(III), sodium hexachlororhodate(III), potassium hexachlororhodate(III), rhodium(III) trichloride, rhodium(III) bromide, rhodium(III) iodide, rhodium(III) sulfate, rhodium(III) nitrate, potassium hexanitrorhodate(III);
said copper catalyst is any one or more of copper acetate hydrate, copper acetate, copper sulfate hydrate, copper sulfate, copper nitrate hydrate, copper nitrate, copper chloride hydrate, copper chloride, copper bromide;
said alkali is any one or more of sodium acetate, sodium carbonate, sodium bicarbonate, potassium acetate, potassium carbonate, potassium bicarbonate, cesium carbonate, lithium carbonate, magnesium acetate, calcium acetate;
the molar ratio of said rhodium catalyst, copper catalyst, alkali, compound **S2,** allenol **5,** and water is (0.005~0.10): (0.005~1.20): (0∼0.60): (1.0∼3.0): 1.0: (0~20.0).

4. The total synthesis method of claim 1, wherein, said total synthesis method comprises the reaction shown in following reaction formula (c): 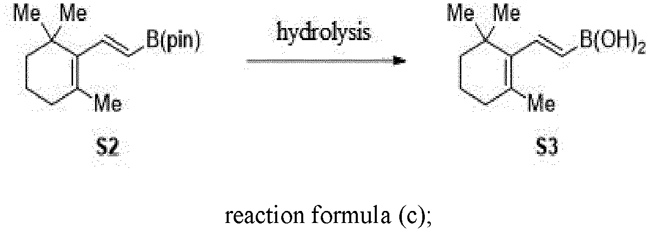 in the reaction shown in said reaction formula (c), compound **S2** is hydrolyzed to produce compound **S3.**

5. The total synthesis method of claim 1, wherein, said total synthesis method comprises the reaction shown in reaction formula (d): 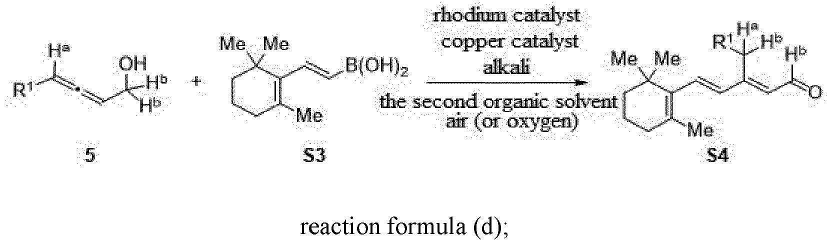 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
in said reaction formula (d), allenol **5** and compound **S3** under the action of rhodium catalyst, copper catalyst, alkali, air or oxygen in the second organic solvent are reacted to produce compound **S4;** said compound **S3** is obtained by the hydrolysis of compound **S2;**
the reaction shown in the reaction formula (d) specifically comprises the following steps:
(1) putting rhodium catalyst, alkali, copper catalyst, compound **S3,** allenol **5** and the second organic solvent into a dry reaction flask, after plugging the reaction flask with a rubber plug, inserting an air balloon or an oxygen balloon to make the reaction flask in an atmosphere of air or oxygen, and stirring the reaction at -20~60 °C for 4∼96 hours;
(2) after the completion of the reaction in said step (1), filtering the mixture in the reaction flask with a short column of silica gel or diatomaceous earth, washing with the third organic solvent, then concentrating and separating to obtain compound **S4;**
said second organic solvent is one or more of tetrahydrofuran, 1,4-dioxane, toluene, acetonitrile, methanol, ethanol, dichloromethane, ether, anisole, methyl tert-butyl ether, ethylene glycol dimethyl ether, ethylene glycol diethyl ether; the amount of said second organic solvent refers to the amount of allenol **5** shown in the reaction formula (d) as the basis, the amount of said second organic solvent is 1.0-20.0 mL/mmol;
said third organic solvent is one or more of ethyl acetate, ether, methanol, ethanol, dichloromethane, tetrahydrofuran, 1,4-dioxane, acetone, acetonitrile; the amount of said third organic solvent refers to the amount of allenol **5** shown in the reaction formula (d) as the basis, the amount of said third organic solvent is 1.0~200 mL/mmol;
said rhodium catalyst is any one or more of dichloro(pentamethylcyclopentadienyl)rhodium(III) dimer, tris(acetonitrile)(pentamethylcyclopentadienyl)rhodium(III) bis(hexafluoroantimonate), rhodium(III) acetylacetonate, pentaamminechlororhodium(III) dichloride, trichlorotris(ethylenediamine)rhodium(III), potassium pentachlororhodate(III), sodium hexachlororhodate(III), potassium hexachlororhodate(III), rhodium(III) trichloride, rhodium(III) bromide, rhodium(III) iodide, rhodium(III) sulfate, rhodium(III) nitrate, potassium hexanitrorhodate(III);
said copper catalyst is any one or more of copper acetate hydrate, copper acetate, copper sulfate hydrate, copper sulfate, copper nitrate hydrate, copper nitrate, copper chloride hydrate, copper chloride, copper bromide;
said alkali is any one or more of sodium acetate, sodium carbonate, sodium bicarbonate, potassium acetate, potassium carbonate, potassium bicarbonate, cesium carbonate, lithium carbonate, magnesium acetate, calcium acetate;
the molar ratio of said rhodium catalyst, copper catalyst, alkali, compound **S3,** allenol **5** is (0.005∼0.10): (0.005~1.20): (0∼0.60): (1.0∼3.0): 1.0.

6. The total synthesis method of claim 1, wherein, said total synthesis method comprises the reaction shown in reaction formula (e): 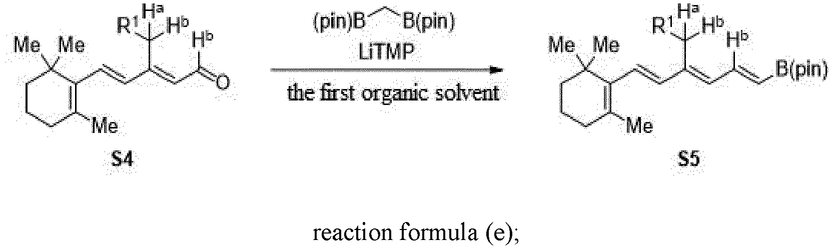 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
in said reaction shown in formula (e), bis[(pinacol)boronyl]methane reacts with compound **S4** under the action of the alkali lithium 2,2,6,6-tetramethylpiperidide LiTMP in the first organic solvent to produce compound **S5;** among them, said lithium 2,2,6,6-tetramethylpiperidide is prepared by using 2,2,6,6-tetramethylpiperidine and N-butyllithium;
said reaction formula (e) specifically comprises the following steps:
(1) protecting the reaction system with inert gas; adding 2,2,6,6-tetramethylpiperidine and the first organic solvent into a dry reaction flask, and adding N-butyllithium reagent at -90~30°C, then continuing stirring for 15 minutes~3 hours at -90~30 °C, preparing lithium 2,2,6,6-tetramethylpiperidide solution on site;
(2) dissolving bis[(pinacol)boronyl]methane in said first organic solvent, which is added to the lithium 2,2,6,6-tetramethylpiperidide solution at -90~30 °C, and then continuing stirring for 1 minute to 2 hours at -90~30 °C;
(3) dissolving compound **S4** in said first organic solvent, which is added to the mixture generated in said step (2) at -90~30 °C, and then stirring the reaction at -90~30 °C for 0.5∼12 hours;
(4) after the completion of the reaction in said step (3), quenching the reaction, and obtaing compound **S5** through extraction, concentration and separation;
said first organic solvent is one or more of tetrahydrofuran, 1,4-dioxane, diethyl ether, anisole, methyl tert-butyl ether, ethylene glycol dimethyl ether, ethylene glycol diethyl ether, and toluene;
the molar ratio of said **S4,** lithium 2,2,6,6-tetramethylpiperidide and bis[(pinacol)boryl]methane is 1:(1.0~2.0):(1.0~2.0), if lithium 2,2,6,6-tetramethylpiperidide solution is prepared on site, the molar ratio of said **S4,** 2,2,6,6-tetramethylpiperidine, n-butyllithium and bis[(pinacol)boryl]methane is 1:(1.0-2.0):(1.0-2.0): (1.0-2.0).

7. The total synthesis method of claim 1, wherein, said total synthesis method comprises the reaction shown in reaction formula (f): 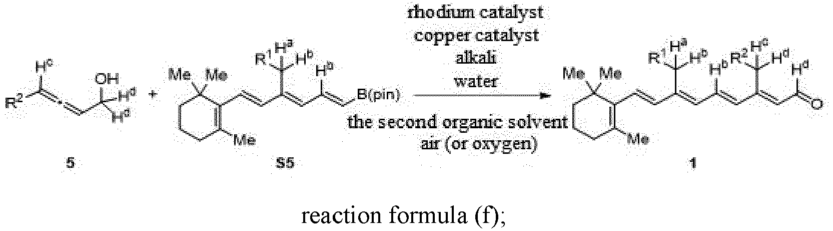 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
in said reaction formula (f), allenol **5** and compound **S5** under the action of rhodium catalyst, copper catalyst, alkali, water, air or oxygen in the second organic solvent are reacted to produce compound **1;**
said reaction shown in the reaction formula (f) specifically comprises the following steps:
(1) putting rhodium catalyst, alkali, copper catalyst, compound **S5,** allenol **5,** water and the second organic solvent into a dry reaction flask, after plugging the reaction flask with a rubber plug, inserting an air balloon or an oxygen balloon to make the reaction flask in an atmosphere of air or oxygen, and stirring the reaction at -20~60 °C for 4∼96 hours;
(2) after the completion of the reaction in said step (1), filtering the mixture in the reaction flask with a short column of silica gel or diatomaceous earth, washing with the third organic solvent, concentrating and separating to obtain compound **1;**
said second organic solvent is one or more of tetrahydrofuran, 1,4-dioxane, toluene, acetonitrile, methanol, ethanol, dichloromethane, ether, anisole, methyl tert-butyl ether, ethylene glycol dimethyl ether, ethylene glycol diethyl ether; the amount of said second organic solvent refers to the amount of allenol **5** shown in the reaction formula (f) as the basis, the amount of said second organic solvent is 1.0-20.0mL/mmol;
said third organic solvent is one or more of ethyl acetate, ether, methanol, ethanol, dichloromethane, tetrahydrofuran, 1,4-dioxane, acetone, acetonitrile; the amount of said third organic solvent refers to the amount of allenol **5** shown in the reaction formula (f) as the basis, the amount of said third organic solvent is 1.0~200mL/mmol;
said rhodium catalyst is any one or more of dichloro(pentamethylcyclopentadienyl)rhodium(III) dimer, tris(acetonitrile)(pentamethylcyclopentadienyl)rhodium(III) bis(hexafluoroantimonate), rhodium(III) acetylacetonate, pentaamminechlororhodium(III) dichloride, trichlorotris(ethylenediamine)rhodium(III), potassium pentachlororhodate(III), sodium hexachlororhodate(III), potassium hexachlororhodate(III), rhodium(III) trichloride, rhodium(III) bromide, rhodium(III) iodide, rhodium(III) sulfate, rhodium(III) nitrate, potassium hexanitrorhodate(III);
said copper catalyst is any one or more of copper acetate hydrate, copper acetate, copper sulfate hydrate, copper sulfate, copper nitrate hydrate, copper nitrate, copper chloride hydrate, copper chloride, copper bromide;
said alkali is any one or more of sodium acetate, sodium carbonate, sodium bicarbonate, potassium acetate, potassium carbonate, potassium bicarbonate, cesium carbonate, lithium carbonate, magnesium acetate, calcium acetate;
the molar ratio of said rhodium catalyst, copper catalyst, alkali, compound **S5,** allenol **5,** and water is (0.005~0.10): (0.005~1.20): (0~0.60): (1.0~3.0): 1.0: (0~20.0).

8. The total synthesis method of claim 1, wherein, said total synthesis method comprises the reaction shown in reaction formula (g): 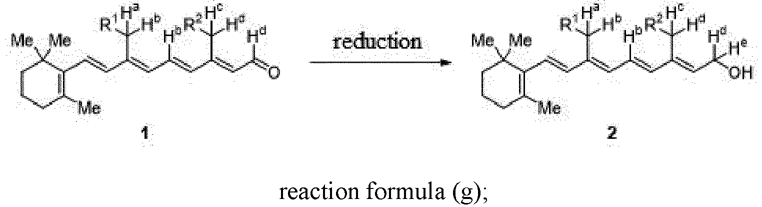 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
H^{e} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; in said hydrocarbyl group with functional group, said functional group include acyl, hydroxyl, halogen, alkoxy, alkoxycarbonyl, formyl, acyloxy, amido, sulfonyl, sulfonyloxy, nitro, carboxy, cyano group;
In said reaction formula (g), compound **1** is subjected to a reduction reaction to produce compound **2.**

9. The total synthesis method of claim 1, wherein, said total synthesis method comprises the reaction as shown in reaction formula (h): 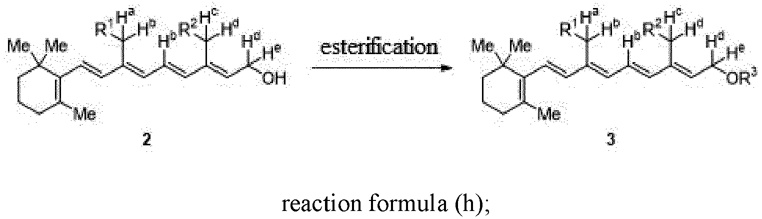 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
H^{e} is hydrogen, deuterium, tritium;
R³ is an acyl group;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; in said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group;
In said reaction formula (h), compound **2** is esterified to produce compound **3.**

10. The total synthesis method of claim 1, wherein, said total synthesis method comprises the reaction as shown in reaction formula (i): 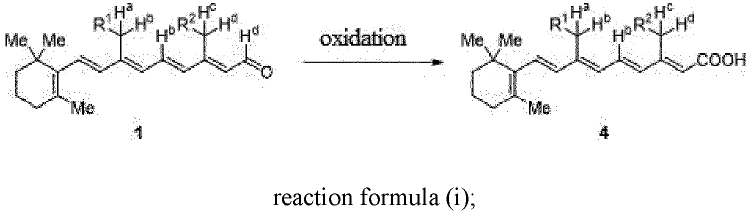 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; in said hydrocarbyl group with functional group, said functional group include acyl, hydroxyl, halogen, alkoxy, alkoxycarbonyl, formyl, acyloxy, amido, sulfonyl, sulfonyloxy, nitro, carboxy, cyano group;
in said reaction formula (i), compound **1** is oxidized to produce compound **4.**

11. Application of the method of claims 1-10 in the preparation of vitamin A and derivative thereof and deuterated compound thereof.

12. Vitamin A and derivative thereof and deuterated compound thereof, wherein, said molecular formula of the compound is as follows: 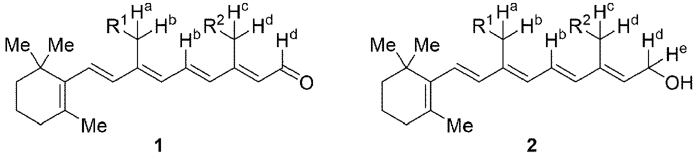 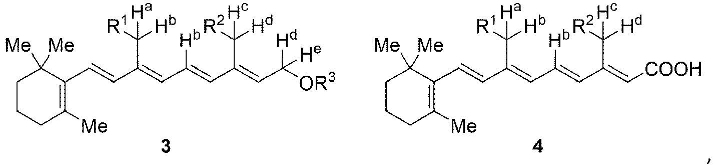 wherein,
H^{a} is hydrogen, deuterium, tritium;
R¹ is hydrogen, deuterium, tritium, phenyl, aryl, heterocyclic, hydrocarbyl, hydrocarbyl group with functional group;
H^{b} is hydrogen, deuterium, tritium;
H^{c} is hydrogen, deuterium, tritium;
R² is hydrogen, deuterium, tritium, phenyl, aryl, heterocyclic, hydrocarbyl, hydrocarbyl group with functional group;
H^{d} is hydrogen, deuterium, tritium;
H^{e} is hydrogen, deuterium, tritium;
R³ is an acyl group;
wherein, said aryl group is a phenyl group with substituent at the ortho, meta and para positions, and said substituent includes alkyl, alkenyl, phenyl, halogen, trifluoromethyl, alkoxy, alkoxycarbonyl, acyl, acyloxy, amido, sulfonyl, sulfonyloxy, hydroxyl, nitro, carboxy, cyano, amino group; said heterocyclic group refers to a 3-10-membered heterocyclic ring whose ring atoms are carbon, nitrogen, oxygen or sulfur, which is an aliphatic ring, an aromatic ring, or a ring formed by combining two or more simple rings; among said hydrocarbyl group with functional group, said functional group includes acyl, hydroxyl, halogen, alkoxy, alkoxycarbonyl, formyl, acyloxy, amido, sulfonyl, sulfonyloxy, nitro, carboxy, cyano group.
